# EUROPEAN PATENT SPECIFICATION

(11) **EP 2 519 173 B1**
(45) Date of publication and mention of the grant of the patent: **23.09.2015**
(21) Application number: 10801084.4
(22) Date of filing: 30.12.2010
(51) Int. Cl.: A61B 18/02, A61B 18/18, A61B 18/00, A61B 18/04, A61B 18/24, A61N 5/10, A61N 7/00

(54) **PATTERNED DENERVATION THERAPY FOR INNERVATED RENAL VASCULATURE**
STRUKTURIERTE DENERVIERUNGSTHERAPIE FÜR INNERVIERTE NIERENGEFÄSSE
THÉRAPIE DE DÉSENSIBILISATION DE CONFIGURATION POUR VASCULATURE RÉNALE INNERVÉE

(30) Priority: 31.12.2009 US 291480 P; 29.12.2010 US 980972
(43) Date of publication of application: 07.11.2012
(73) Proprietor: Boston Scientific Scimed, Inc., Maple Grove, MN 55311 (US)
(72) Inventor: VRBA, Anthony, Maple Grove MN 55311 (US); SOGARD, Dave, Edina MN 55424 (US); RIZQ, Raed, Maple Grove MN 55311 (US); ALLOCCO, Dominic, Edina MN 55424 (US); GERDTS, Michael, Big Lake MN 55309 (US); KOCUR, Gordon, Lino Lakes MN 55014 (US); KANGAS, Steve, Woodbury MN 55125 (US)
(74) Representative: Vossius & Partner Patentanwälte Rechtsanwälte mbB
(86) International application number: PCT/US2010/062458
(87) International publication number: WO 2011/082279

(56) References cited:
- EP-A1- 2 204 134
- WO-A2-2006/041881
- US-A1- 2007 129 720
- US-A1- 2009 221 939
- US-A1- 2009 306 644

## Description

### TECHNICAL FIELD

The present invention is related to systems for improving cardiac and/or renal function through neuromodulation, including disruption and termination of renal nerve activity.

### BACKGROUND

The kidneys are instrumental in a number of body processes, including blood filtration, regulation of fluid balance, blood pressure control, electrolyte balance, and hormone production. One primary function of the kidneys is to remove toxins, mineral salts, and water from the blood to form urine. The kidneys receive about 20-25% of cardiac output through the renal arteries that branch left and right from the abdominal aorta, entering each kidney at the concave surface of the kidneys, the renal hilum.

Blood flows into the kidneys through the renal artery and the afferent arteriole, entering the filtration portion of the kidney, the renal corpuscle. The renal corpuscle is composed of the glomerulus, a thicket of capillaries, surrounded by a fluid-filled, cup-like sac called Bowman's capsule. Solutes in the blood are filtered through the very thin capillary walls of the glomerulus due to the pressure gradient that exists between the blood in the capillaries and the fluid in the Bowman's capsule. The pressure gradient is controlled by the contraction or dilation of the arterioles. After filtration occurs, the filtered blood moves through the efferent arteriole and the peritubular capillaries, converging in the interlobular veins, and finally exiting the kidney through the renal vein.

Particles and fluid filtered from the blood move from the Bowman's capsule through a number of tubules to a collecting duct. Urine is formed in the collecting duct and then exits through the ureter and bladder. The tubules are surrounded by the peritubular capillaries (containing the filtered blood). As the filtrate moves through the tubules and toward the collecting duct, nutrients, water, and electrolytes, such as sodium and chloride, are reabsorbed into the blood.

The kidneys are innervated by the renal plexus which emanates primarily from the aorticorenal ganglion. Renal ganglia are formed by the nerves of the renal plexus as the nerves follow along the course of the renal artery and into the kidney. The renal nerves are part of the autonomic nervous system which includes sympathetic and parasympathetic components. The sympathetic nervous system is known to be the system that provides the bodies "fight or flight" response, whereas the parasympathetic nervous system provides the "rest and digest" response. Stimulation of sympathetic nerve activity triggers the sympathetic response which causes the kidneys to increase production of hormones that increase vasoconstriction and fluid retention. This process is referred to as the renin-angiotensin-aldosterone-system (RAAS) response to increased renal sympathetic nerve activity.

In response to a reduction in blood volume, the kidneys secrete renin, which stimulates the production of angiotensin. Angiotensin causes blood vessels to constrict, resulting in increased blood pressure, and also stimulates the secretion of the hormone aldosterone from the adrenal cortex. Aldosterone causes the tubules of the kidneys to increase the reabsorption of sodium and water, which increases the volume of fluid in the body and blood pressure.

Congestive heart failure (CHF) is a condition that has been linked to kidney function. CHF occurs when the heart is unable to pump blood effectively throughout the body. When blood flow drops, renal function degrades because of insufficient perfusion of the blood within the renal corpuscles. The decreased blood flow to the kidneys triggers an increase in sympathetic nervous system activity (i.e., the RAAS becomes too active) that causes the kidneys to secrete hormones that increase fluid retention and vasorestriction. Fluid retention and vasorestriction in turn increases the peripheral resistance of the circulatory system, placing an even greater load on the heart, which diminishes blood flow further. If the deterioration in cardiac and renal functioning continues, eventually the body becomes overwhelmed, and an episode of heart failure decompensation occurs, often leading to hospitalization of the patient.

Hypertension is a chronic medical condition in which the blood pressure is elevated. Persistent hypertension is a significant risk factor associated with a variety of adverse medical conditions, including heart attacks, heart failure, arterial aneurysms, and strokes. Persistent hypertension is a leading cause of chronic renal failure. Hyperactivity of the sympathetic nervous system serving the kidneys is associated with hypertension and its progression. Deactivation of nerves in the kidneys via renal denervation can reduce blood pressure, and may be a viable treatment option for many patients with hypertension who do not respond to conventional drugs.

The U.S. patent applications No. 2007/0129720 A1 and 2009/0221939 A1 disclose systems for thermally-induced renal neuromodulation.

### SUMMARY

Systems of the present invention are directed to modifying renal sympathetic nerve activity using various forms of denervation therapy.

The invention is as defined in the appended claims.

Embodiments of the present invention are directed to a renal denervation apparatus that includes a treatment element dimensioned for deployment on or within a renal artery of a patient. The treatment element comprises a predefined pattern arranged to complete at least one revolution of the treatment element. The apparatus includes a treatment source. The treatment source and treatment element are configured to cooperatively facilitate communication of an agent from the treatment source to the pattern arrangement of the treatment element. The treatment element is configured to deliver denervation therapy using the agent via the pattern arrangement to one or more regions of the renal artery adjacent the pattern arrangement, such that at least one complete revolution of the renal artery is subjected to the denervation therapy.

In some embodiments, the treatment element is dimensioned for intravascular deployment within the renal artery. In other embodiments, the treatment element is dimensioned for extravascular deployment at the renal artery.

In various embodiments, the treatment source includes a cryogen source and the agent comprises a cryogen. In some embodiments, the treatment source includes a thermal transfer fluid source and the agent comprises a thermal transfer fluid heated to a predefined temperature above body temperature.

In accordance with various embodiments, a catheter system of the present invention includes a catheter comprising a flexible shaft having a proximal end, a distal end, a length, and a lumen arrangement extending between the proximal and distal ends. The length of the shaft is sufficient to access a patient's renal artery relative to a percutaneous access location of the patient. A treatment element is provided at the distal end of the shaft and fluidly coupled to the lumen arrangement. The treatment element is dimensioned for deployment on or within the renal artery and comprises a predefined pattern arranged to complete at least one revolution of the treatment element. The treatment element is configured to receive a thermal transfer fluid from the lumen arrangement and deliver thermal denervation therapy using the thermal transfer fluid via the pattern arrangement to one or more regions of the renal artery adjacent the pattern arrangement, such that at least one complete revolution of the renal artery is subjected to the thermal denervation therapy.

In some embodiments, the catheter includes a hinge mechanism provided on the shaft proximal of the treatment element. The hinge mechanism may include a slotted tube arrangement, for example. The hinge mechanism is configured to facilitate preferential bending at the distal end of the shaft to aid in directing the treatment element into the renal artery from the abdominal aorta. A biasing mechanism may be included and configured to generate a biasing force sufficient to effect engagement between the treatment element and the inner wall of the renal artery.

The pattern arrangement of the treatment element may be formed as an integral part of the distal end of the shaft or as a pre-fabricated sleeve mounted at the distal end of the shaft. In some embodiments, a cryoballoon is fluidly coupled to the lumen arrangement of the shaft and the lumen arrangement is configured to receive the cryoballoon. Pressurizing the cryoballoon expands the pattern arrangement at the distal end of the shaft and causes the pattern arrangement to contact or come into close proximity of an inner wall of the renal artery. The distal end of the shaft is preferably formed from a resilient material that facilitates expansion and contraction of the shaft responsive to expansion and contraction of the cryoballoon. In this configuration, the cryoballoon may define a non-compliant balloon.

According to other embodiments, the distal end of the catheter's shaft is formed from a material having a stiffness greater than a stiffness of the cryoballoon, and the relative stiffness of the shaft's distal end and the cryoballoon material allows portions of the cryoballoon's exterior surface exposed by apertures of the pattern arrangement to expand into the apertures and contact the inner wall of the renal artery. In this configuration, the cryoballoon may define a compliant balloon.

In further embodiments, the treatment element includes a cryoballoon fluidly coupled to the lumen arrangement of the shaft, and the pattern arrangement is formed as an integral part of the cryoballoon or is formed as a separate sleeve that is affixed to an outer surface of the cryoballoon.

In some embodiments, the pattern arrangement comprises a predetermined pattern of apertures. In other embodiments, the pattern arrangement comprises a predetermined pattern of thermally conductive elements or regions. The pattern arrangement may be contiguous or non-contiguous, and may form a generally spiral or helical pattern.

According to some embodiments, the thermal transfer fluid comprises a cryogen having a boiling temperature at ambient pressure of about 0°C or lower. In other embodiments, the thermal transfer fluid comprises a cryogen having a boiling temperature at ambient pressure of about -60°C or lower. In further embodiments, the thermal transfer fluid comprises a cryogen having a boiling temperature at ambient pressure of about -140°C or lower. In other embodiments, the thermal transfer fluid comprises a cryogen having a boiling temperature at ambient pressure of about -180°C or lower.

The above summary of the present invention is not intended to describe each embodiment or every implementation of the present invention. Advantages and attainments, together with a more complete understanding of the invention, will become apparent and appreciated by referring to the following detailed description and claims taken in conjunction with the accompanying drawings.
Figure 1 is an illustration of a right kidney and renal vasculature including a renal artery branching laterally from the abdominal aorta;
Figures 2A and 2B illustrate sympathetic innervation of the renal artery;
Figure 3A illustrates various tissue layers of the wall of the renal artery;
Figures 3B and 3C illustrate a portion of a renal nerve;
Figure 4 illustrates a medical system configured for treating a target vessel, such as the renal artery, for purposes of modifying sympathetic nerve activity along the vessel in accordance with intravascular embodiments of the present invention;
Figure 5 illustrates a medical system configured for treating a target vessel, such as the renal artery, for purposes of modifying sympathetic nerve activity along the vessel in accordance with extravascular embodiments of the present invention;
Figure 6 shows a medical system configured for cryogenically treating a target vessel, such as the renal artery, for purposes of modifying sympathetic nerve activity along the vessel in accordance with embodiments of the present invention;
Figure 7 shows a cross section of the cryothermal catheter apparatus shown in Figure 6;
Figure 8 shows another embodiment of medical system configured to provide extravascular access to a target vessel, such as the renal artery, and deliver denervation therapy to vessel nerve fibers from the outer wall of the target vessel in accordance with embodiments of the present invention;
Figures 9A-9E illustrate embodiments of a delivery apparatus that incorporates a hinge mechanism for facilitating intravascular access to the renal artery from the abdominal aorta in accordance with embodiments of the present invention;
Figures 10A-10G illustrate various embodiments of a treatment element that comprises a patterned treatment surface in accordance with embodiments of the present invention;
Figures 11A-11B illustrate various embodiments of a treatment element provided at a distal end of a catheter apparatus and comprising a patterned treatment surface in accordance with embodiments of the present invention;
Figures 12A and 12B illustrate embodiments of a treatment element provided at a distal end of a catheter apparatus and comprising a patterned treatment surface in accordance with embodiments of the present invention according to the present invention;
Figures 13A and 13B illustrate embodiments of a treatment element that includes dual balloon arrangements in accordance with embodiments of the present invention;
Figures 14A and 14B illustrate a treatment element that includes dual balloon arrangements in accordance with embodiments of the present invention;
Figures 15A and 15B show a treatment element that includes dual balloon arrangements and a pre-stressed feature incorporated into an outer balloon in accordance with embodiments of the present invention;
Figures 16A-16E are cross sections of a treatment element in accordance with various embodiments of the present invention;
Figures 17A-18B show different embodiments of a multiple lumen catheter configured to deliver denervation therapy to a target vessel in accordance with the present invention; and
Figures 19A-19B are cross sections showing various layers of a treatment element in accordance with the present invention.

While the invention is amenable to various modifications and alternative forms, specifics thereof have been shown by way of example in the drawings and will be described in detail. It is to be understood, however, that the intention is not to limit the invention to the particular embodiments described. On the contrary, the intention is to cover all modifications, equivalents, and alternatives falling within the scope of the invention as defined by the appended claims.

### DETAILED DESCRIPTION

In the following description, references are made to the accompanying drawings which illustrate various embodiments of the invention. It is to be understood that other embodiments may be utilized, and structural and functional changes may be made to these embodiments without departing from the scope of the present invention.

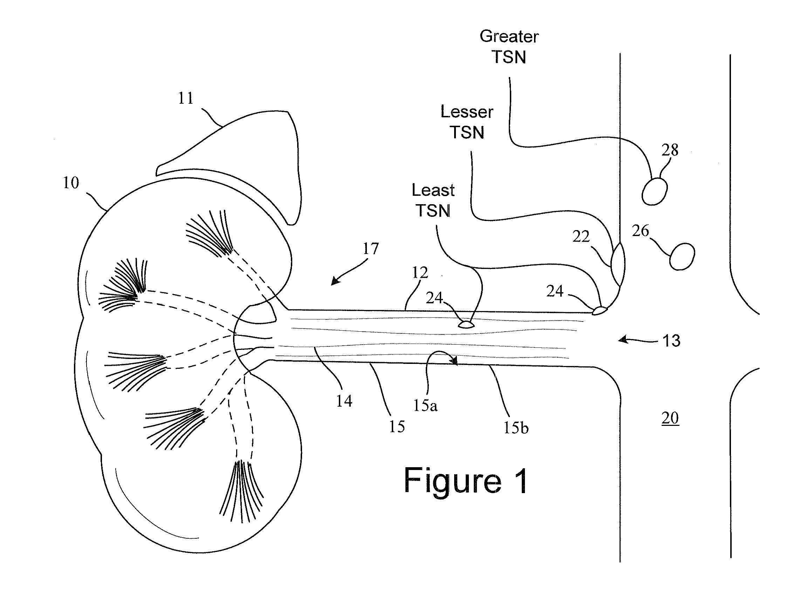

Figure 1 is an illustration of a right kidney 10 and renal vasculature including a renal artery 12 branching laterally from the abdominal aorta 20. In Figure 1, only the right kidney 10 is shown for purposes of simplicity of explanation, but reference will be made herein to both right and left kidneys and associated renal vasculature and nervous system structures, all of which are contemplated within the context of embodiments of the present invention. The renal artery 12 is purposefully shown to be disproportionately larger than the right kidney 10 and abdominal aorta 20 in order to facilitate discussion of various features and embodiments of the present disclosure.

The right and left kidneys are supplied with blood from the right and left renal arteries that branch from respective right and left lateral surfaces of the abdominal aorta 20. Each of the right and left renal arteries is directed across the crus of the diaphragm, so as to form nearly a right angle with the abdominal aorta 20. The right and left renal arteries extend generally from the abdominal aorta 20 to respective renal sinuses proximate the hilum 17 of the kidneys, and branch into segmental arteries and then interlobular arteries within the kidney 10. The interlobular arteries radiate outward, penetrating the renal capsule and extending through the renal columns between the renal pyramids. Typically, the kidneys receive about 20% of total cardiac output which, for normal persons, represents about 1200 mL of blood flow through the kidneys per minute.

The primary function of the kidneys is to maintain water and electrolyte balance for the body by controlling the production and concentration of urine. In producing urine, the kidneys excrete wastes such as urea and ammonium. The kidneys also control reabsorption of glucose and amino acids, and are important in the production of hormones including vitamin D, renin and erythropoietin.

An important secondary function of the kidneys is to control metabolic homeostasis of the body. Controlling hemostatic functions include regulating electrolytes, acid-base balance, and blood pressure. For example, the kidneys are responsible for regulating blood volume and pressure by adjusting volume of water lost in the urine and releasing erythropoietin and renin, for example. The kidneys also regulate plasma ion concentrations (e.g., sodium, potassium, chloride ions, and calcium ion levels) by controlling the quantities lost in the urine and the synthesis of calcitrol. Other hemostatic functions controlled by the kidneys include stabilizing blood pH by controlling loss of hydrogen and bicarbonate ions in the urine, conserving valuable nutrients by preventing their excretion, and assisting the liver with detoxification.

Also shown in Figure 1 is the right suprarenal gland 11, commonly referred to as the right adrenal gland. The suprarenal gland 11 is a star-shaped endocrine gland that rests on top of the kidney 10. The primary function of the suprarenal glands (left and right) is to regulate the stress response of the body through the synthesis of corticosteroids and catecholamines, including cortisol and adrenaline (epinephrine), respectively. Encompassing the kidneys 10, suprarenal glands 11, renal vessels 12, and adjacent perirenal fat is the renal fascia, e.g., Gerota's fascia, (not shown), which is a fascial pouch derived from extraperitoneal connective tissue.

The autonomic nervous system of the body controls involuntary actions of the smooth muscles in blood vessels, the digestive system, heart, and glands. The autonomic nervous system is divided into the sympathetic nervous system and the parasympathetic nervous system. In general terms, the parasympathetic nervous system prepares the body for rest by lowering heart rate, lowering blood pressure, and stimulating digestion. The sympathetic nervous system effectuates the body's fight-or-flight response by increasing heart rate, increasing blood pressure, and increasing metabolism.

In the autonomic nervous system, fibers originating from the central nervous system and extending to the various ganglia are referred to as preganglionic fibers, while those extending from the ganglia to the effector organ are referred to as postganglionic fibers. Activation of the sympathetic nervous system is effected through the release of adrenaline (epinephrine) and to a lesser extent norepinephrine from the suprarenal glands 11. This release of adrenaline is triggered by the neurotransmitter acetylcholine released from preganglionic sympathetic nerves.

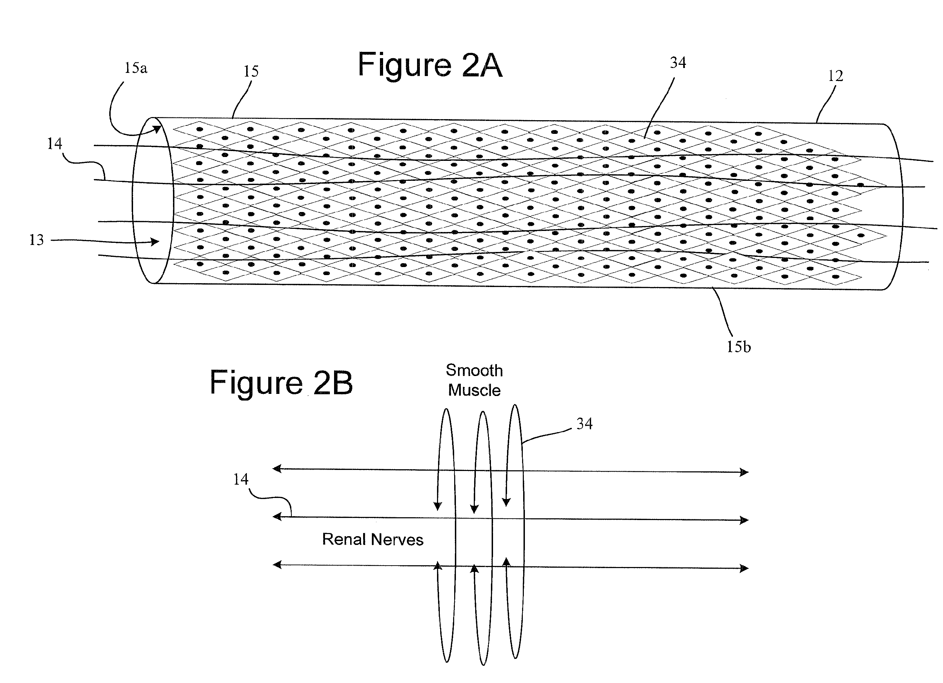

The kidneys and ureters (not shown) are innervated by the renal nerves 14. Figures 1 and 2A-2B illustrate sympathetic innervation of the renal vasculature, primarily innervation of the renal artery 12. The primary functions of sympathetic innervation of the renal vasculature include regulation of renal blood flow and pressure, stimulation of renin release, and direct stimulation of water and sodium ion reabsorption.

Most of the nerves innervating the renal vasculature are sympathetic postganglionic fibers arising from the superior mesenteric ganglion 26. The renal nerves 14 extend generally axially along the renal arteries 12, enter the kidneys 10 at the hilum 17, follow the branches of the renal arteries 12 within the kidney 10, and extend to individual nephrons. Other renal ganglia, such as the renal ganglia 24, superior mesenteric ganglion 26, the left and right aorticorenal ganglia 22, and celiac ganglia 28 also innervate the renal vasculature. The celiac ganglion 28 is joined by the greater thoracic splanchnic nerve (greater TSN). The aorticorenal ganglia 26 is joined by the lesser thoracic splanchnic nerve (lesser TSN) and innervates the greater part of the renal plexus.

Sympathetic signals to the kidney 10 are communicated via innervated renal vasculature that originates primarily at spinal segments T10-T12 and L1. Parasympathetic signals originate primarily at spinal segments S2-S4 and from the medulla oblongata of the lower brain. Sympathetic nerve traffic travels through the sympathetic trunk ganglia, where some may synapse, while others synapse at the aorticorenal ganglion 22 (via the lesser thoracic splanchnic nerve, i.e., lesser TSN) and the renal ganglion 24 (via the least thoracic splanchnic nerve, i.e., least TSN). The postsynaptic sympathetic signals then travel along nerves 14 of the renal artery 12 to the kidney 10. Presynaptic parasympathetic signals travel to sites near the kidney 10 before they synapse on or near the kidney 10.

With particular reference to Figure 2A, the renal artery 12, as with most arteries and arterioles, is lined with smooth muscle 34 that controls the diameter of the renal artery lumen 13. Smooth muscle, in general, is an involuntary non-striated muscle found within the media layer of large and small arteries and veins, as well as various organs. The glomeruli of the kidneys, for example, contain a smooth muscle-like cell called the mesangial cell. Smooth muscle is fundamentally different from skeletal muscle and cardiac muscle in terms of structure, function, excitation-contraction coupling, and mechanism of contraction.

Smooth muscle cells can be stimulated to contract or relax by the autonomic nervous system, but can also react on stimuli from neighboring cells and in response to hormones and blood borne electrolytes and agents (e.g., vasodilators or vasoconstrictors). Specialized smooth muscle cells within the afferent arteriole of the juxtaglomerular apparatus of kidney 10, for example, produces renin which activates the angiotension II system.

The renal nerves 14 innervate the smooth muscle 34 of the renal artery wall 15 and extend lengthwise in a generally axial or longitudinal manner along the renal artery wall 15. The smooth muscle 34 surrounds the renal artery circumferentially, and extends lengthwise in a direction generally transverse to the longitudinal orientation of the renal nerves 14, as is depicted in Figure 2B.

The smooth muscle 34 of the renal artery 12 is under involuntary control of the autonomic nervous system. An increase in sympathetic activity, for example, tends to contract the smooth muscle 34, which reduces the diameter of the renal artery lumen 13 and decreases blood perfusion. A decrease in sympathetic activity tends to cause the smooth muscle 34 to relax, resulting in vessel dilation and an increase in the renal artery lumen diameter and blood perfusion. Conversely, increased parasympathetic activity tends to relax the smooth muscle 34, while decreased parasympathetic activity tends to cause smooth muscle contraction.

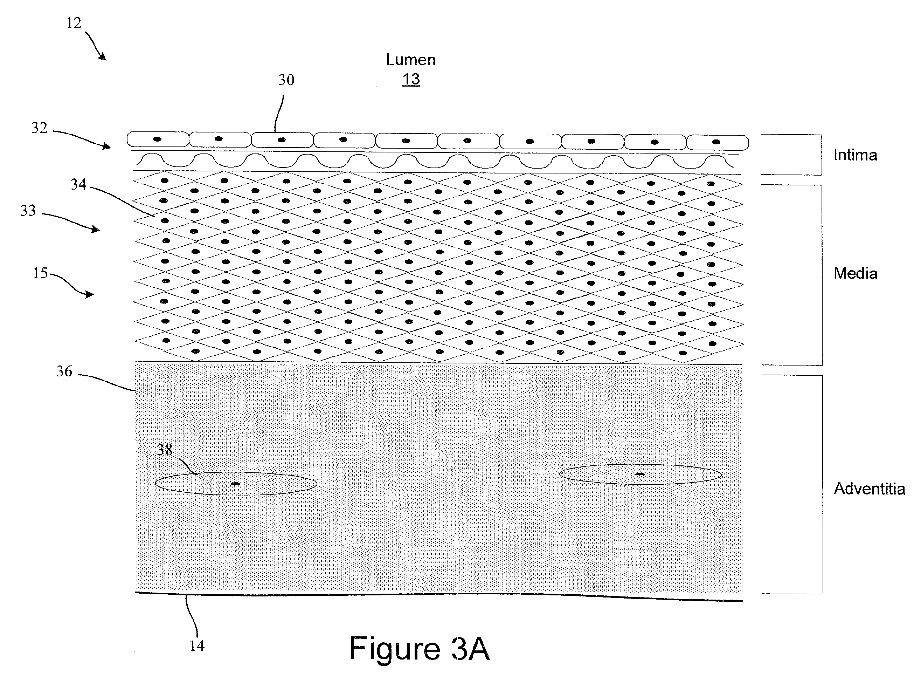

Figure 3A shows a segment of a longitudinal cross-section through a renal artery, and illustrates various tissue layers of the wall 15 of the renal artery 12. The innermost layer of the renal artery 12 is the endothelium 30, which is the innermost layer of the intima 32 and is supported by an internal elastic membrane. The endothelium 30 is a single layer of cells that contacts the blood flowing though the vessel lumen 13. Endothelium cells are typically polygonal, oval, or fusiform, and have very distinct round or oval nuclei. Cells of the endothelium 30 are involved in several vascular functions, including control of blood pressure by way of vasoconstriction and vasodilation, blood clotting, and acting as a barrier layer between contents within the lumen 13 and surrounding tissue, such as the membrane of the intima 32 separating the intima 32 from the media 34, and the adventitia 36. The membrane or maceration of the intima 32 is a fine, transparent, colorless structure which is highly elastic, and commonly has a longitudinal corrugated pattern.

Adjacent the intima 32 is the media 33, which is the middle layer of the renal artery 12. The media is made up of smooth muscle 34 and elastic tissue. The media 33 can be readily identified by its color and by the transverse arrangement of its fibers. More particularly, the media 33 consists principally of bundles of smooth muscle fibers 34 arranged in a thin plate-like manner or lamellae and disposed circularly around the arterial wall 15. The outermost layer of the renal artery wall 15 is the adventitia 36, which is made up of connective tissue. The adventitia 36 includes fibroblast cells 38 that play an important role in wound healing. A renal nerve 14 is shown proximate the adventitia 36 and extending longitudinally along the renal artery 12. The main trunk of the renal nerves 14 generally lies in or on the adventitia of the renal artery, with certain branches coursing into the media to enervate the renal artery smooth muscle.

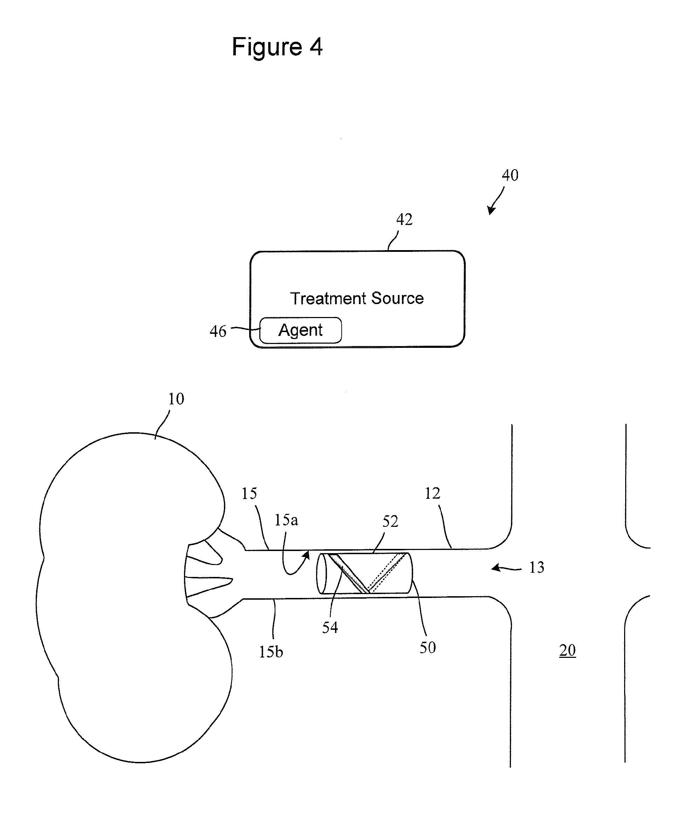

Turning now to Figure 4, there is illustrated a medical system 40 configured for treating a target vessel for purposes of modifying sympathetic nerve activity along the vessel in accordance with embodiments of the present invention. The representative embodiment of the medical system 40 shown in Figure 4 is configured for intravascular treatment of the renal artery 12 to terminate renal sympathetic nerve activity. Preferably, embodiments of the medical system 40 according to Figure 4 are configured for treating the renal artery 12 to irreversibly terminate all renal sympathetic nerve activity.

System embodiments according to Figure 4 include a treatment source 40 and a treatment element 50 dimensioned for intravascular placement within the renal artery 12. The treatment element 50 comprises a patterned treatment surface 52. The treatment source 40 and the treatment element 50 are configured to cooperatively facilitate communication of an agent 46 from the treatment source 40 to the patterned treatment surface 52 of the treatment element 50. The patterned treatment surface 52 and agent 46 are configured to cooperatively treat the renal artery wall 15 via the interior renal artery wall 15a to terminate all renal sympathetic nerve activity. A variety of intravascular delivery techniques may be used to place the treatment element 50 within the lumen 13 of the renal artery 12.

The treatment surface 52 of the treatment element 50 includes a patterned arrangement 54 that has a longitudinal aspect and a circumferential aspect. The patterned arrangement 54 is configured to complete at least one revolution of the treatment element 50. The patterned arrangement 54 may be contiguous or non-contiguous. For example, a contiguous patterned arrangement 54 preferably has a spiral or other shape that completes at least one revolution of the treatment element 50 (e.g., at least one turn of the spiral or helix). A non-contiguous or segmented patterned arrangement 54 preferably comprises a multiplicity of spaced-apart patterned segments that collectively complete at least one revolution of the treatment element 50. A contiguous or segmented patterned arrangement 54 can take on a variety of configurations, and need not be limited to spiral or helical shapes.

The patterned arrangement 54 may be integral to the treatment element 50 or may be a separate structure. The patterned arrangement 54 may comprise one or more surface structures or treatment features, surface discontinuities, voids or apertures, or combinations of these and other features. The patterned arrangement 54 may comprise disparate regions that vary in terms of one or more of material, thermal, electrical, optical, chemical, mechanical, pharmacological, and dimensional properties or characteristics. For example, the patterned arrangement 54 may comprise regions differing in terms of thickness, permeability, porosity, density, elasticity, thermal conductivity, and electrical conductance, among others.

In some embodiments, the patterned arrangement 54 may be disposed or expressed on the surface of the treatment element 50, such that the agent 46 is communicated directly to or through the inner wall 15a of the renal artery 12. In other embodiments, the patterned arrangement 54 may be disposed or expressed over the treatment element 50, such that the agent 46 is communicated through the patterned arrangement 54 and to or through the inner wall 15 a of the renal artery 12.

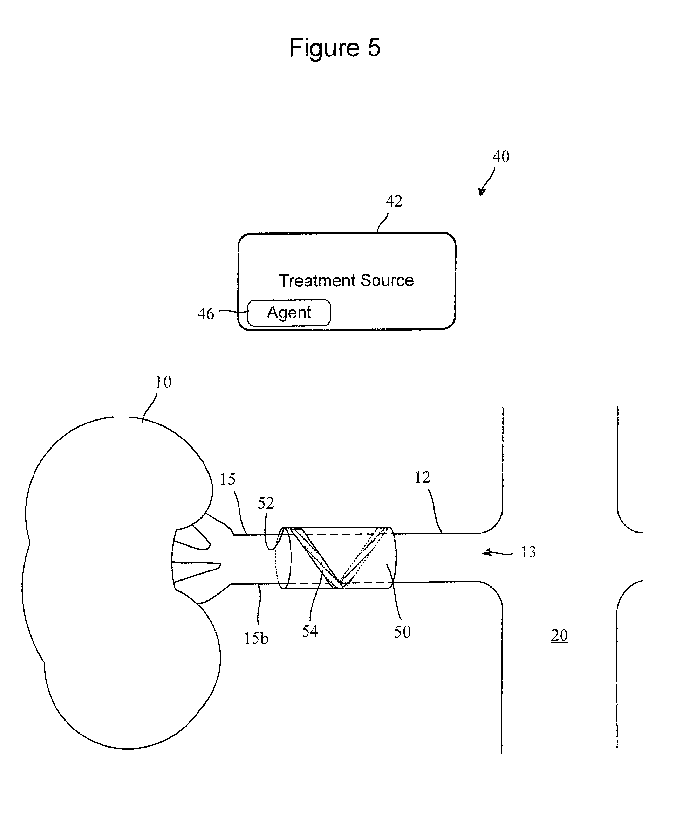

Figure 5 shows an embodiment of a treatment element 50 dimensioned for extravascular placement on the renal artery 12. A percutaneous intrathoracic access procedure, such as a laparoscopic, thoracoscopic, or other minimally invasive surgical procedure, is preferably used to place the treatment element 50 on the outer wall 15b of the renal artery 12. The treatment element 50, according to the embodiment of Figure 5, comprises a patterned treatment surface 52 situated adjacent an outer portion 15b of the renal artery wall 15. The treatment source 40 and the treatment element 50 are configured to cooperatively facilitate communication of an agent 46 from the treatment source 40 to the patterned treatment surface 52 of the treatment element 50. The patterned treatment surface 52 and agent 46 are configured to cooperatively treat the renal artery wall 15 via the outer renal artery wall 15b to terminate all renal sympathetic nerve activity.

The treatment surface 52 of the treatment element 50 includes a patterned arrangement 54 of a type discussed hereinabove. As discussed above, the patterned arrangement 54 has a longitudinal aspect and a circumferential aspect, may be contiguous or non-contiguous, and preferably completes at least one revolution of the treatment element 50.

In some embodiments, the patterned arrangement 54 may be disposed or expressed on the surface of the treatment element 50, such that the agent 46 is communicated directly to or at least partially through the outer wall 15a of the renal artery 12 (e.g., direct thermal treatment). In other embodiments, the patterned arrangement 54 may be disposed or expressed over or within the treatment element 50, such that the agent 46 is communicated through the patterned arrangement 54 and to or at least partially through the outer wall 15b of the renal artery 12 (e.g., thermal treatment delivered through apertures or a permeable layer). The agent 26 may take the form of thermal energy that is coupled to the patterned arrangement 54, such as by an energy source located outside the body.

A patterned treatment element 50 of the present invention advantageously facilitates a "one-shot" denervation therapy of the renal artery or other vessel in accordance with embodiments of the present invention. The term "one-shot" treatment refers to treating the entirety of a desired portion of a vessel without having to move the treatment implement or arrangement to other vessel locations in order to complete the treatment procedure (as is the case for a step-and-repeat denervation therapy approach). A one-shot treatment approach of the present invention advantageously facilitates delivery of denervation therapy that treats at least one location of each nerve fiber extending along a target vessel, such as the renal artery, without having to reposition the treatment element 50 during denervation therapy delivery. Embodiments of the present invention allow a physician to position a treatment element 50 at a desired vessel location, and completely treat the vessel without having to move the treatment element 50 to a new vessel location. It is to be understood that devices and methods that utilize a patterned treatment element 50 provide advantages and benefits other than facilitating one-shot treatment of a vessel, and that treatment element patterning that enables one-shot vessel treatment is not a required feature in all embodiments of the present invention.

It can be appreciated that the type of agent 46 will vary in accordance with the particulars of the treatment source 42 and treatment element 50. The agent 46 takes the form of a thermal transfer fluid (hot or cold).

The treatment source 42 may be external to the body, implantable (temporarily or chronically), or comprise external and implantable elements. In some embodiments, the treatment source 42 is physically connected to the treatment element 50, and the agent 46 is communicated to the treatment element 52 via the connection. In other embodiments, the treatment source 42 is physically separate from the treatment element 50, and the agent 46 is communicated or coupled to the treatment element 50 by means other than a physical connection with the treatment element 50. In further embodiments, different agents 46 and means for communicating or coupling the agent 46 to the treatment element 50 may be employed.

A biasing mechanism may be employed to maintain engagement between the treatment element 50 and the inner wall 15a (for intravascular embodiments) or the outer wall 15b (for extravascular embodiments) of the renal artery 12. Various biasing mechanisms may be employed, including single and multiple balloon arrangements, multi-lumen catheters, combinations of balloons and catheters, spring arrangements, elastic arrangements, clamp or cuff arrangements, stent or cage structures, mechanical interlinking arrangements, and shape-memory elements, among others. The biasing mechanism and/or the treatment element 50 may include a radio-opaque material or include radio-opaque markers to facilitate fluoroscopic visualization.

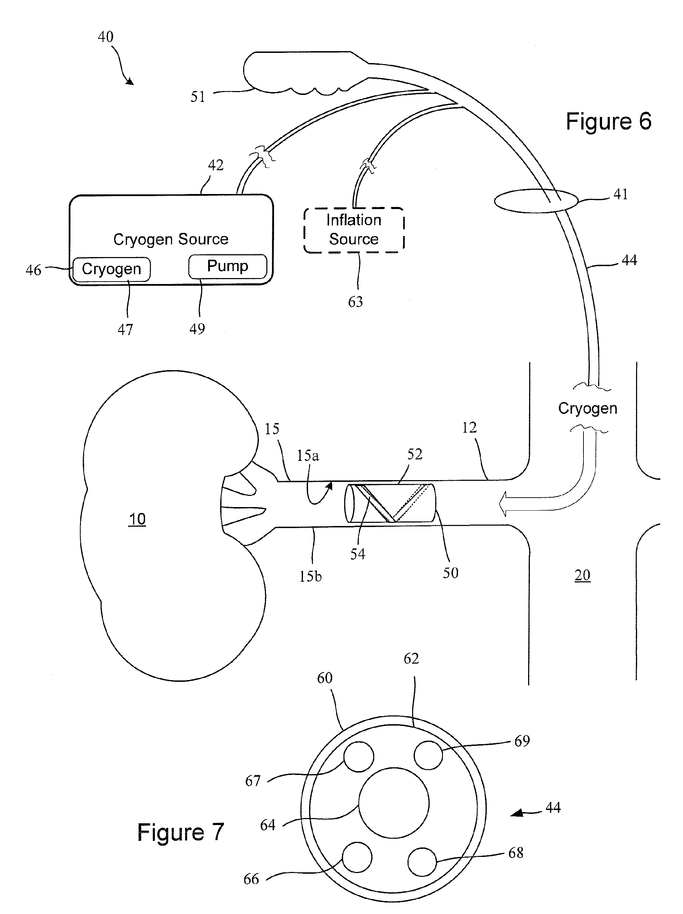

Figure 6 shows a medical system 40 configured for cryogenically treating a renal artery for purposes of modifying renal sympathetic nerve activity along the artery wall in accordance with embodiments of the present invention. The medical system 40 is preferably configured for cryogenically treating the renal artery to irreversibly terminate all renal sympathetic nerve activity along the artery wall.

The medical system 40 shown in Figure 6 is configured to facilitate intravascular access to the renal artery 12 and deliver renal denervation therapy to or through the inner wall 15a of the renal artery 12. A cryogen source 42 includes a reservoir 47 fluidly coupled to a pump 49. A cryogen 46 is contained within the reservoir 47. A variety of cryogens 26 may be employed, including cold saline or cold saline and ethanol mixture, Freon or other fluorocarbon refrigerants, nitrous oxide, liquid nitrogen, and liquid carbon dioxide, for example.

The cryogen source 42 is coupled to a cryothermal catheter apparatus 44, which may be a unitary or multi-component apparatus. Figure 7 shows a cross section of the cryothermal catheter apparatus 44, which is described in greater detail hereinbelow. The catheter apparatus 44 may include a tube (e.g., a cryoprobe), lumen, and/or balloon arrangement through which the cryogen 46 is communicated between the cryogen source 42 and an intravascular cryoablation element 50 disposed at the distal end of the catheter apparatus 44.

In some embodiments, the cryogen 46, when released inside the cryoablation element 50 (e.g., a cryoballoon), undergoes a phase change that cools the treatment portion of the cryoablation element 50 by absorbing the latent heat of vaporization from the tissue surrounding the cryoablation element 50, and by cooling of the vaporized gas as it enters a region of lower pressure inside the cryoablation element 50 (the Joule-Thomson effect).

As a result of the phase change and the Joule-Thompson effect, heat is extracted from the surroundings of the cryoablation element 50, thereby cooling the treatment portion of the cryoablation element 50 and renal tissue that is in contact with the treatment portion of the cryoablation element 50. The gas released inside the cryoablation element 50 may be exhausted through a separate exhaust lumen provided in the catheter 44. The pressure inside the cryoablation element 50 may be controlled by regulating one or both of a rate at which cryogen 46 is delivered and a rate at which the exhaust gas is extracted.

It has been shown experimentally that at sufficiently low temperatures, the blood in contact with the cryoablation element's treatment portion will freeze, thereby acting as a thermally conducting medium to conduct heat away from adjacent blood and tissue of the renal artery 12. The diameters and insulating properties of the cryoablation element 50 can be designed such that the middle region of the renal artery 12 is a primary target for treatment. Cryogenically treating the middle region of the renal artery 12 reduces the adverse impact on the distal and proximal portions of the renal artery 12. For example, the cryoablation element 50 can be designed such that only partial contact with the renal artery wall is permitted and insulating material is placed elsewhere in order to reduce and control the region(s) that are subject to cryotherapy.

The catheter apparatus 44 is preferably lined with or otherwise incorporates insulation material(s) 60 having appropriate thermal and mechanical characteristics suitable for a selected cryogen. A lumen arrangement is shown in Figure 7 that can include one or a number of lumens depending on the particular implementation of the catheter apparatus 44. The lumen arrangement of Figure 7 is shown for illustrative purposes only, and is not intended to limit the configuration and/or functionality of the catheter apparatus 44. Accordingly, various lumens shown in Figure 7 need not be incorporated in a given catheter apparatus 44. Alternatively, lumens other than those shown in Figure 7 may be incorporated in a given catheter apparatus 44, including lumens formed on the exterior wall of the catheter's shaft.

In some embodiments, the lumen arrangement includes a first lumen 66, for supplying a cryogen to the distal end of the catheter apparatus 44, and a second lumen 68, for returning the cryogen to the proximal end of the catheter apparatus 44. The supply and return lumens 66, 68 may be coupled to a cryotube or cryoballoon disposed at the distal end of the catheter apparatus 44. The cryogen may be circulated through the cryotube or cryoballoon via a hydraulic circuit that includes the cryogen source 42, supply and return lumens 66, 68, and the cryotube or cryoballoon disposed at the distal end of the catheter apparatus 44.

In configurations that incorporate a cryoballoon, the supply lumen 66 may be supplied with a pressurized cryogen by the cryogen source 42 that both pressurizes the cryoballoon and provides the cryogen to the cryoablation element 50. In configurations that incorporate a cryotube, the catheter apparatus 44 may include one or more inflation lumens (e.g., lumens 67 and/or 69) that fluidly communicate with one or more balloons disposed at the distal end of the catheter apparatus 44. In further embodiments, one or more cryoballoons and one or more biasing balloons may be incorporated at the distal end of the catheter apparatus 44, with appropriate supply, return, and pressurization lumens provided to fluidly communicate with the cryogen source 42 and an optional inflation fluid source 63.

Embodiments of the present invention may incorporate selected balloon, catheter, lumen, control, and other features of the devices disclosed in the following commonly owned U.S. patents and published patent applications: U.S. Patent Publication Nos. 2009/0299356, 2009/0299355, 2009/0287202, 2009/0281533, 2009/0209951, 2009/0209949, 2009/0171333, 2009/0171333, 2008/0312644, 2008/0208182, 2008/0058791 and 2005/0197668, and U.S. Patent Nos. 5868735, 6290696, 6648878, 6666858,6709431,6929639,6989009,7022120,7101368,7172589,7189227,and 7220257, 6355029, 6428534, 6432102, 6468297, 6514245, 6602246, 6648879, 6786900, 6786901, 6811550, 6908462, 6972015, and 7081112.

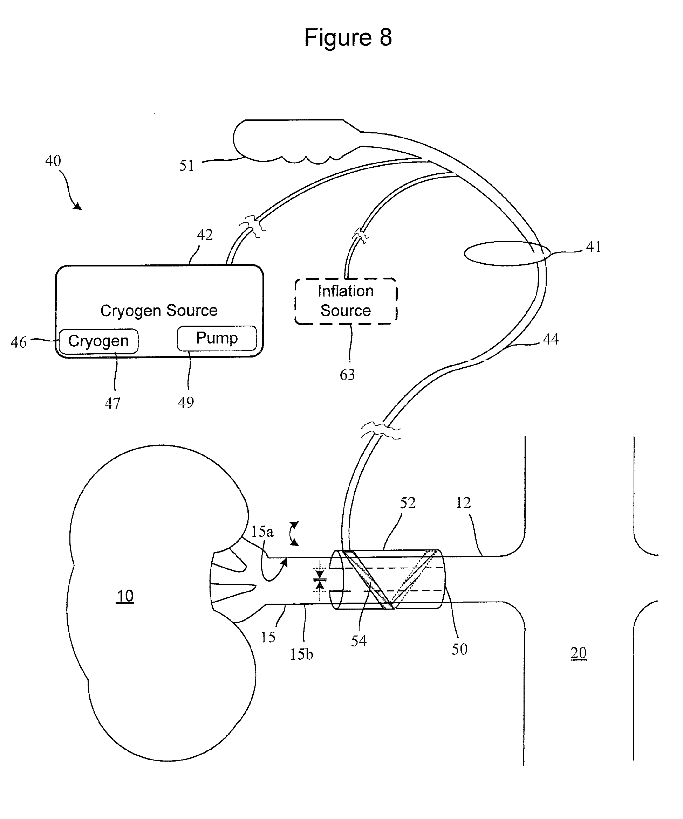

Figure 8 shows another embodiment of medical system 40 configured to provide extravascular access to the renal artery 12 and deliver renal denervation therapy to the renal nerve from the outer wall 15b of the renal artery 12. Because the renal nerve fibers are situated on or proximate the outer wall 15b of the renal artery 12, extravascular denervation therapy of the renal artery 12 advantageously minimizes the amount of tissue subjected to the cryogen or other agent relative to an intravascular approach.

According to the embodiment shown in Figure 8, the cryoablation element 50 is percutaneously introduced into the body and advanced to the renal artery 12 via a subcutaneous intrathoracic path by use of an appropriate surgical approach and instrument, such as a trocar or cannula. As previously discussed, laparoscopic, thoracoscopic, or other minimally invasive surgical procedure represent preferred surgical approaches. The cryoablation element 50 incorporates a coupling mechanism that enables the physician to position the cryoablation element 50 on the outer wall 15b of the renal artery 12 so that at least the ablation pattern 54 completes at least one turn or revolution about the periphery of the artery 12.

The cryoablation element 50 may incorporate a cuff mechanism that can be manipulated so that opposing edges of the cuff contact each other. The cuff coupling mechanism shown in Figure 8 represents a circumferential or annular cuff implementation. The cryoablation element 50 may have other coupling mechanisms, such as a spiral or helical shaped coupling mechanism, among other configurations. The coupling mechanism may be integral to the cryoablation element 50, such as by incorporation of an interlocking arrangement disposed along the all or a portion of the opposing edges of the cryoablation element cuff (e.g., a latching arrangement). A spiral or helical coupling arrangement may provide for *in situ* coupling of the cryoablation element 50 to the outer wall 15b of the renal artery 12, such as by wrapping a spiral or helical shape memory portion of the cryoablation element 50 around the renal artery 12. Cuff embodiments in accordance with the present invention may be implemented to include features of various known vascular and nerve cuff structures, such as those disclosed in U.S. Pat. Nos. 7,584,004; 6,106,477; 5,251,634; and 4,649,936; and in U.S. Patent Publication No. 2008/0004673.

With further reference to Figures 6-8, the catheter apparatus 44 may incorporate a proximal section that includes a control mechanism 51 to facilitate physician manipulation of the catheter apparatus 44. In certain embodiments, the control mechanism 51 facilitates physician manipulation of the cryoablation element 50, such as delivery and/or deployment of the cryoablation element 50 within or on the renal artery 12. In some configurations, the control mechanism 51 may include a steerable portion that facilitates physician control of rotation and longitudinal displacement of the catheter apparatus 44 and/or the cryoablation element 50 through the access vasculature and into the renal artery 12.

In other configurations, the catheter apparatus 44 includes a guide lumen 64 through which a guide wire may be advanced. The guide wire may be advanced through a vascular access port into the femoral artery to the aorta 20 and into the renal artery 12. The catheter apparatus 44 may then be advanced over the guide wire using an over-the-wire navigation technique. The control mechanism 51 may include a steerable portion that facilitates physician control of orientation and longitudinal displacement of the guide wire and/or the catheter apparatus 44 during navigation to, and cannulation of, the renal artery 12. A marker band may be attached or applied to the guide wire and/or the catheter apparatus 44 so that the position of the guide wire and/or the catheter apparatus 44 in the patient's body may be visualized using known imaging techniques.

The control mechanism 51 may accommodate a number of physician tools that are external of a patient's body when in use, and allow the physician to perform various functions at the distal section of the catheter apparatus 44. Each of the tools may be coupled to one or more associated lumens in the catheter apparatus 44 using one or more manifolds at the proximal section, for example.

As will be discussed in greater detail hereinbelow, some embodiments incorporate a biasing or anchoring mechanism disposed at the distal end of the catheter apparatus 44, such as at the cryoablation element 50. The type and configuration of the biasing or anchoring mechanism varies depending on whether the cryoablation element 50 is configured for intravascular or extravascular positioning. As previously discussed, suitable biasing mechanisms include single and multiple balloon arrangements, multi-lumen catheters, combinations of balloons and catheters, spring arrangements, elastic arrangements, clamp or cuff arrangements, stent or cage structures, mechanical interlinking arrangements, shape-memory elements, and other mechanisms or structures that are capable of creating a biasing force sufficient to facilitate engagement between the cryoablation element 50 and the inner or outer vessel wall 15a, 15b. For example, a saline solution or a cryogen may be forced into a balloon or inflatable catheter structure that mechanically cooperates with the cryoablation element 50 to cause expansion of the balloon or catheter structure. Other inflation fluids may also be used, including liquids and gases.

Accessing each of the right and left renal arteries from the abdominal aorta 20 is often a challenge, since each of the renal arteries form nearly a right angle with respect to the abdominal aorta 20. Embodiments of the invention are directed to intravascular delivery apparatuses that enhance access to, and cannulation of, the renal arteries via the superior or inferior abdominal aorta 12.

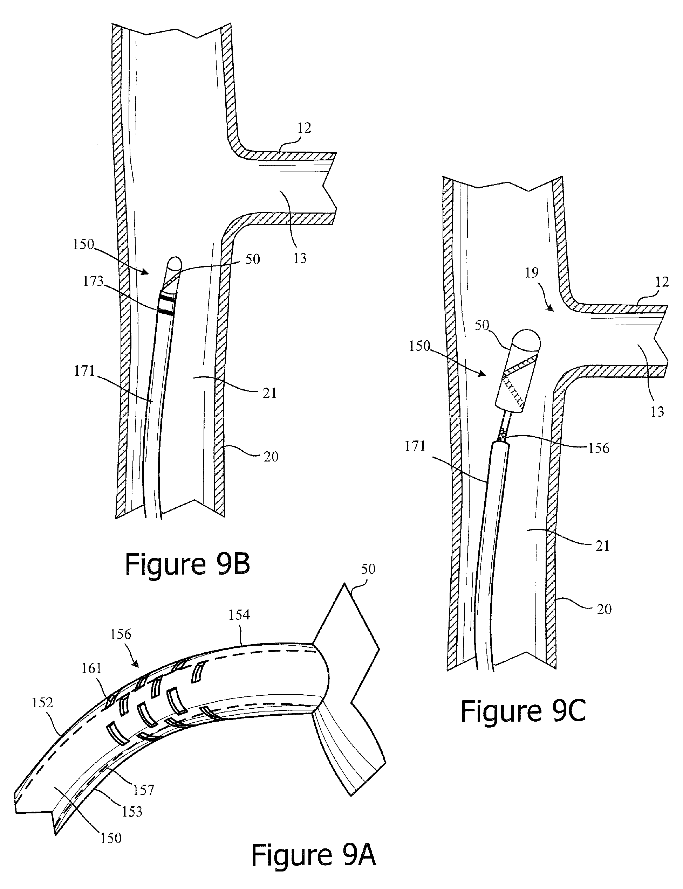

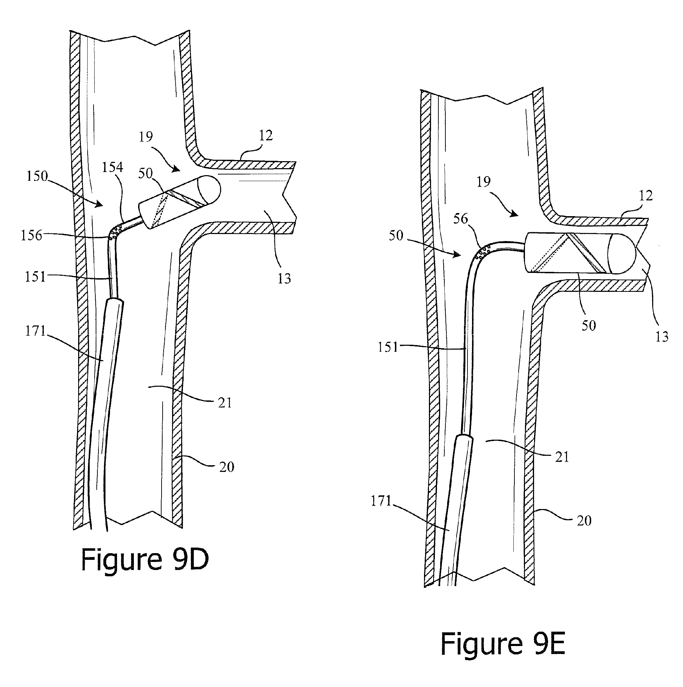

Figures 9A-9E show a hinge mechanism 156 built into a treatment catheter 150 proximate a treatment element 50. The treatment element 50 is shown as a generic component in Figures 9A-9E, and is intended to represent any of the treatment elements described herein. The hinge mechanism 156 is constructed to enhance user manipulation of the treatment element 50 when navigating the treatment element 50 around a nearly 90 degree turn from the abdominal aorta 20 into the ostium 19 of the renal artery 12. Integration of a hinge mechanism 156 into the treatment element 50 advantageously reduces the force that the treatment element 50 may impart to the renal artery 12 during the freeze/thaw cycle.

Figure 9A illustrates a portion of the treatment catheter 150 that incorporates a hinge mechanism 156 in accordance with embodiments of the invention. The hinge mechanism 156 is provided at a location of the catheter 150 between a proximal section 152 and a distal section 154 of the catheter shaft 151. The hinge mechanism 156 is preferably situated near the proximal section of the treatment element 50. According to various embodiments, the hinge mechanism 156 comprises a slotted tube arrangement that is configured to provide a flexible hinge point of the catheter shaft 151 proximate the treatment element 50.

The catheter shaft 151 may be formed to include an elongate core member 157 and a tubular member 153 disposed about a portion of the core member 157. The tubular member 153 may have a plurality of slots 161 formed therein. The slotted hinge region of the catheter shaft 151 may be configured to have a preferential bending direction.

For example, and as shown in Figure 9A, tubular member 152 may have a plurality of slots 161 that are formed by making a pair of cuts into the wall of tubular member 161 that originate from opposite sides of tubular member 153, producing a lattice region of greater flexibility relative to the proximal and distal sections 151, 154 of the catheter shaft 151. The thickness of the catheter wall at the hinge region 156 can be varied so that one side of the catheter wall is thicker than the opposite side. This difference in wall thickness alone or in combination with a difference in slot (void) density at the hinge region 156 provides for a preferential bending direction of the distal portion of the treatment catheter 150.

A hinge arrangement 156 constructed to provide for a preferential bending direction allows a physician to more easily and safely navigate the treatment element 50 to make the near 90 degree turn into the renal artery from the abdominal aorta 20. One or more marker bands may be incorporated at the hinge region 156 to provide visualization of this region of the catheter shaft 151 during deployment. Details of useful hinge arrangements that can be incorporated into embodiments of a treatment catheter 150 of the present invention are disclosed in U.S. Patent Publication Nos. 2008/0021408 and 2009/0043372. It is noted that the treatment catheter 150 may incorporate a steering mechanism in addition to, or exclusion of, a hinge arrangement 156. Known steering mechanisms incorporated into steerable guide catheters may be incorporated in various embodiments of a treatment catheter 150 of the present invention.

Figures 9B-9E illustrate a series of views of a treatment catheter 150 of the present invention at different states of deployment within a patient. A typical deployment procedure involves percutaneous delivery of a guide catheter 171 to an access vessel (e.g., a vascular access port into the femoral artery), via an introducer sheath (not shown), and advancement of the guide catheter 171 through access vasculature to the abdominal aorta 20 at a location inferior (or superior) to the renal artery 12. The guide catheter 171 preferably includes one or more marker bands 173 to aid in visualization of at least the distal open tip of the guide catheter 171. The guide catheter 171 may include a steering mechanism, of a type discussed above.

With the guide catheter 171 positioned near the ostium 19 of the renal artery 12, the treatment element 50, typically in a collapsed configuration, is advanced through the lumen of the guide catheter 171. Marker bands may be provided on or near the treatment element 50 to facilitate visualization of the treatment element 50 when being advanced through the guide catheter 171. As is shown in Figure 9E, the treatment element 50 is advanced out of the guide catheter 171, typically allowing the treatment element 50 to expand somewhat upon exiting the distal open tip of the guide catheter 171. As the region of the catheter shaft 151 comprising the hinge mechanism 156 passes out of the guide catheter 171, the distal portion 154 of the catheter shaft 151 preferably bends relative to the proximal portion 152 of the catheter shaft 151 in a direction dictated by the preferential bend provided by the hinge mechanism 156. The catheter shaft 151 may be rotated by the physician to achieve proper orientation of the treatment element 50 relative to the ostium 19 of the renal artery 12. Further advancement of the treatment element 50 (or retraction of the guide catheter 171) relative to the guide catheter 171 allows for an increase in bend angle at the hinge region 156, allowing the physician to safely advance the distal tip of the treatment element 50 into the ostium 19 of the renal artery lumen 13.

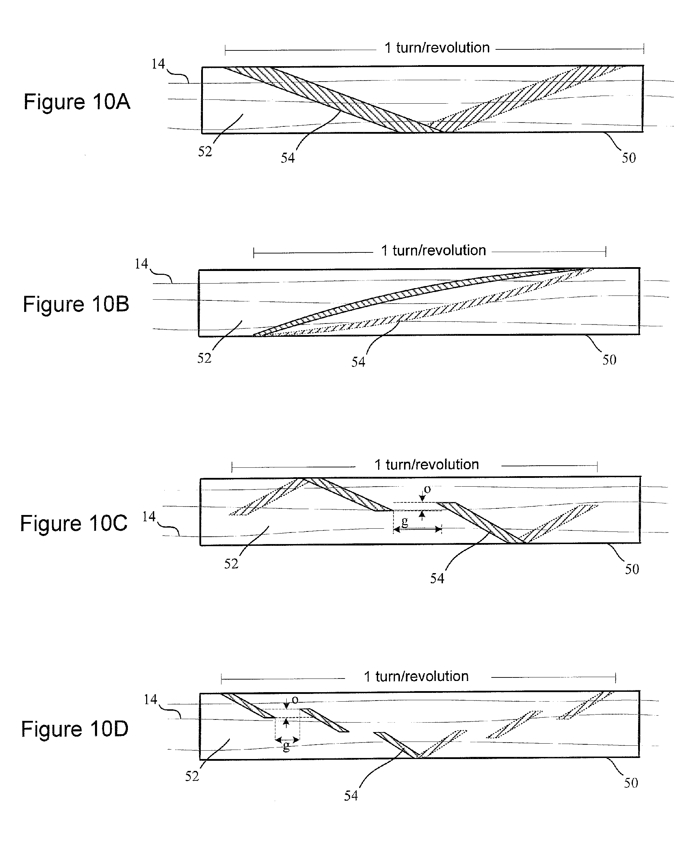

Turning now to Figures 10A-10G, various embodiments of a treatment element 50 are illustrated that comprise a patterned treatment surface 52 according to the present invention. The patterned surfaces 54 of the treatment elements 50 shown in Figures 10A-10G represent a non-limiting, non-exhaustive set of possible pattern configurations that may be implemented in accordance with embodiments of the present invention. Embodiments of the present invention include unitary treatment elements 50 that incorporate a patterned treatment surface 52 and multiple component treatment elements 50 that cooperate to provide a treatment surface 52 having a desired pattern 54.

Embodiments of the treatment element 50 include those configured for intravascular placement (e.g., endoluminal), extravascular placement (e.g., on or proximate the outer wall of a vessel), or both intra- and extravascular placement. A treatment agent may be delivered to the patterned surface 54 via an intravascular source (e.g., via a catheter or balloon), an extravascular source (e.g., via a catheter), or a body-external source (e.g., via an energy source external to the body). In some embodiments, the patterned surface 54 of the treatment element 50 is disposed on, or expressed at, the outer surface of the treatment element 50. In other embodiments, the patterned surface 54 of the treatment element 50 is fashioned to incorporate voids, apertures, or thin wall portions (e.g., permeable or non-permeable) that take on a desired pattern. According to the invention, portions of the treatment surface 52 adjacent to, or otherwise devoid of, the treatment pattern 54 include material or features that provide enhanced thermal insulation to protect tissue surrounding the target tissue to be treated. A treatment agent may be delivered through the voids, apertures, or thin wall portions of the treatment element 50.

Useful treatment elements 50 include those that impart nerve injury to the renal vasculature in accordance with a desired shape that at least disrupts, and preferably irreversibly terminates, renal sympathetic nerve activity. Particularly useful treatment elements 50 include those that can impart such nerve injury to the renal artery using a one-shot denervation therapy approach. In other words, a particularly useful treatment element 50 is one that facilitates delivery of denervation therapy that treats at least one location of each renal nerve fiber 14 extending along the renal artery 12 without having to reposition the treatment element 50 during denervation therapy delivery. It is noted that renal nerve fibers 14 are depicted in Figures 10A-10G for illustrative purposes and to facilitate an enhanced understanding of aspects of the embodiments shown in these and other figures.

Figure 10A shows an embodiment of a treatment element 50 that incorporates a generally spiral or helical treatment pattern 54. The spiral or helical treatment pattern 54 is shown to complete at least one revolution or turn of the treatment element 50, which ensures that at least one location of each renal nerve fiber 14 extending along the renal artery 12 is subject to denervation therapy. Figure 10B shows another embodiment of a treatment element 50 that incorporates a generally elliptically shaped treatment pattern 54. The elliptically shaped treatment pattern 54 may vary in terms of eccentricity and tilt relative to the radial or longitudinal axis of the treatment element 50. The elliptically shaped treatment pattern 54 is shown to complete at least one revolution or turn of the treatment element 50.

Figure 10C shows an embodiment of a treatment element 50 that incorporates a segmented treatment pattern 54 that defines a generally spiral or helical shape. The segmented treatment pattern 54 is shown to include two spiral or helical portions separated by a longitudinal gap, g. A circumferential overlap, *o*, may be provided between the end of the first spiral portion and the beginning of the second spiral portion to prevent inclusion or formation of a circumferential gap therebetween. The segmented spiral or helical treatment pattern 54 of Figure 10C is shown to collectively complete at least one revolution or turn of the treatment element 50, ensuring that at least one location of each renal nerve fiber 14 extending along the renal artery 12 is subject to denervation therapy.

Figure 10D shows an embodiment of a treatment element 50 that incorporates a multiplicity of treatment pattern segments that collectively define a generally spiral or helical shaped pattern 54. Individual spiral treatment pattern segments shown in Figure 10D are separated by a longitudinal gap, g. A circumferential overlap, *o*, may be provided between adjacent pattern segments. The segmented spiral or helical treatment pattern 54 of Figure 10D is shown to complete at least one revolution or turn of the treatment element 50. It is noted that individual segments of a segmented treatment pattern can be distributed in any configuration along the longitudinal axis of the treatment element 50, as long as the individual segments collectively have a surface coverage sufficient to complete at least one revolution or turn of the treatment element 50, such that at least one location of each renal nerve fiber 14 extending along the renal artery 12 is subject to denervation therapy.

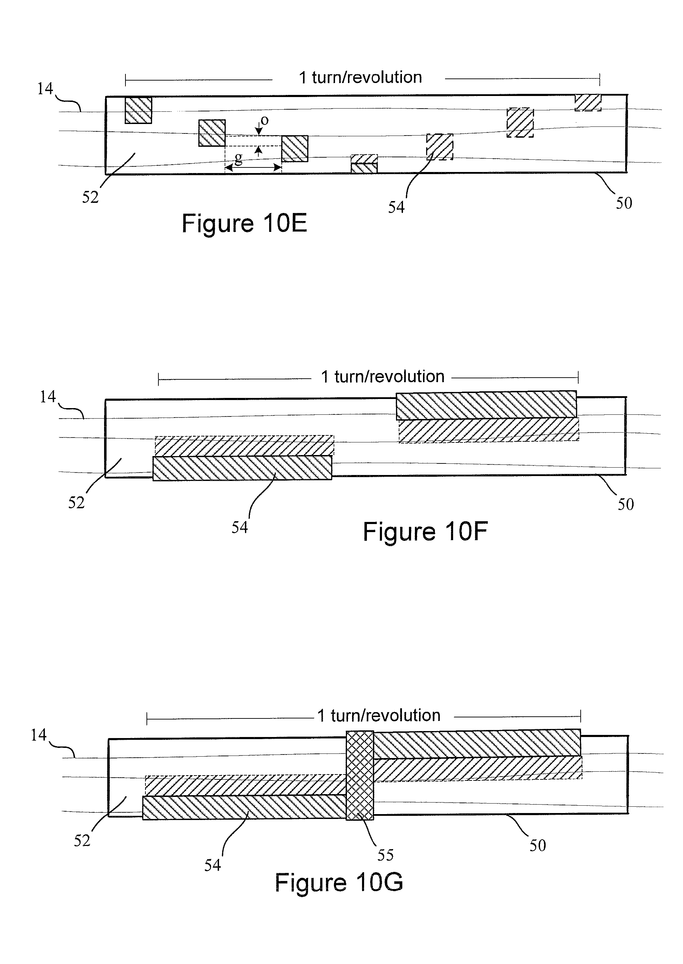

Figure 10E shows an embodiment of a treatment element 50 that incorporates a segmented treatment pattern 54 that defines an overall generally spiral or helical shape. The segmented treatment pattern 54 is shown to include a series of longitudinally spaced square or rectangular portions separated by a longitudinal gap, g. A circumferential overlap, *o*, may be provided between adjacent square or rectangular portions to prevent inclusion or formation of a circumferential gap between individual square or rectangular portions. The segmented pattern 54 of Figure 10E comprising the series of square or rectangular portions is shown to collectively complete at least one revolution or turn of the treatment element 50. It is understood that the pattern segments shown in Figure 10E may be of any geometry (e.g., any polygonal shape, such as a triangle, a quadrilateral, a pentagon, etc.).

Figure 10F shows an embodiment of a treatment element 50 that incorporates two U- or C-shaped treatment pattern segments that together define a segmented treatment pattern 54 of the present invention. The two U- or C-shaped treatment pattern segments shown in Figure 10F are separated by a longitudinal gap, and may overlap one another by a slight amount to ensure that at least one location of each renal nerve fiber 14 extending along the renal artery 12 is subject to denervation therapy.

Figure 10G shows an embodiment of a treatment element 50 that incorporates two U- or C-shaped treatment pattern segments each connected to an annular element 55. The two U- or C-shaped treatment pattern segments shown in Figure 10F are separated by a longitudinal gap, and together complete at least one revolution or turn of the treatment element 50. The annular element 55 may be a polymeric or metallic member that lends structural support or integrity to the treatment pattern elements and/or the basic structure of the treatment element 50. The annular element 55 may be fashioned from a thermally and/or electrically conductive material or a thermally and/or electrically insulating material. The annular element 55 may incorporate a manifold fluidly coupled to a fluid carrying arrangement of the treatment pattern elements 54. The manifold of the annular element 55 may incorporate a port arrangement configured to receive a fluidic treatment agent from a catheter or other conduit coupled to the annular element 55 via an intravascular or extravascular access path.

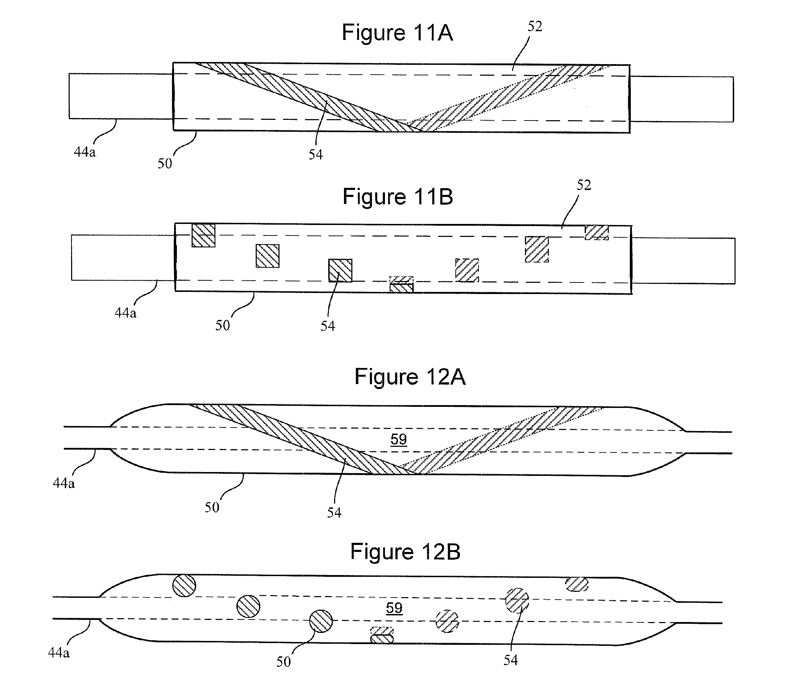

Referring now to Figures 11A-13B, there is illustrated various embodiments of a treatment element 50 provided at a distal end of a catheter apparatus and comprising a patterned treatment surface 52 according to the present invention. In Figures 11A and 11B, a treatment element 50 having a predefined treatment pattern 54 is shown disposed at the distal end 44a of a catheter. The treatment element 50 is preferably a sleeve or sheath that incorporates the predefined treatment pattern 54. The treatment element sleeve or sheath 50 may be fashioned as a component separate from the catheter and subsequently fixed to the distal end 44a of the catheter. Alternatively, the treatment element sleeve or sheath 50 may be formed as an integral element of the distal end 44a of the catheter. The patterned arrangement 54 may comprise one or more surface structures or treatment features, surface discontinuities, voids or apertures, or combinations of these and other features, as discussed previously.

According to some embodiments, one or both of the treatment element 50 and the distal end 44a of the catheter may incorporate a biasing or anchoring mechanism, such as an expandable structure, stent, cage, elastic arrangement, or interlinking mesh arrangement, for example, which is capable of expanding or otherwise creating a biasing force sufficient to facilitate engagement between the treatment element 50 and the inner wall of a vessel.

Figures 12A and 12B illustrate embodiments of a treatment element 50 provided at a distal end 44a of a catheter apparatus and comprising a patterned treatment surface 52 according to the present invention. The treatment elements 50 shown in Figures 12A and 12B each comprise a balloon arrangement 59 that incorporates a predefined treatment pattern 54. The treatment pattern 54 of the balloon arrangement 59 may be fashioned as a separate component from the balloon arrangement 59 and subsequently fixed thereto (e.g., a patterned sleeve or sheath) or formed as in integral element of the balloon arrangement 59. The patterned arrangement 54 of the balloon arrangement 59 may comprise one or more surface structures or treatment features, surface discontinuities, voids or apertures, or combinations of these and other features, as discussed previously.

The balloon arrangement 59 shown in Figures 12A and 12B may comprise a single balloon structure or a multiplicity of balloon structures. One or more inflation lumens may be provided in the catheter supporting the balloon arrangement 59 that fluidly couple the inflation lumens to an external inflation and/or cryogen source. A saline solution and/or a cryogen may be forced into and from the balloon arrangement 59 to controllably inflate and deflate one or more of the expandable structures of the balloon arrangement 59. A cryogen, whether used for pressurization of the balloon arrangement 59 or not, is fluidly communicated to the treatment pattern 54 to deliver denervation therapy to the target vessel when the balloon arrangement 59 is in its pressurized state.

According to the invention, the outer surface of the balloon arrangement 59 incorporates material with a relatively low thermal conductivity (e.g., thermally insulating material) that forms the main body of the balloon arrangement 59. The treatment pattern 54 or pattern segments 54 are formed from relatively high thermally conductive material. In other embodiments, an inner layer of the balloon arrangement 59 may incorporate a polymeric composite material with a low thermal conductivity, and the outer portion of the balloon arrangement 59 may incorporate a patterned or apertured layer comprising a polymeric composite material with a low thermal conductivity. In such embodiments, regions of the inner layer with high thermal conductivity are exposed for thermally treating the inner wall of a target vessel through apertures of the outer layer with low thermal conductivity.

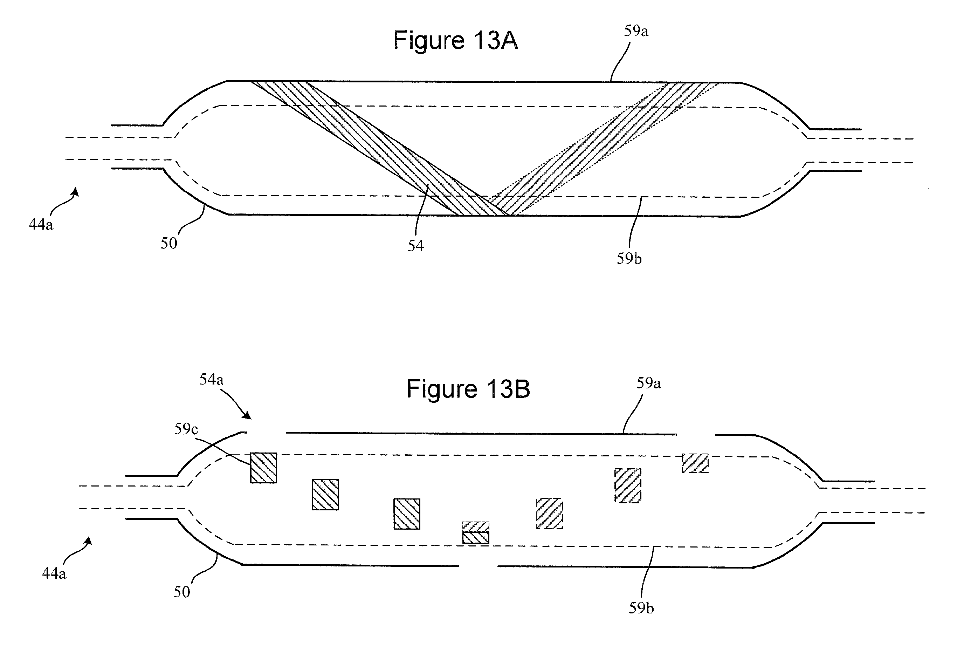

Figures 13A and 13B illustrate embodiments of a treatment element 50 that includes dual balloon arrangements 59a, 59b. In some embodiments, as shown in Figure 13A, an outer balloon 59a of the treatment element 50 incorporates a treatment pattern 54 configured to facilitate delivery of a denervation therapy directly to or through the inner wall of a vessel. An inner balloon 59b serves as a biasing balloon that, when inflated, expands and forces at least the treatment pattern arrangement 54 of the outer balloon 59a against the inner wall of the target vessel. The inner balloon 59b may be controllably pressurized using saline or other passive fluid. A cryogen is communicated to the treatment pattern arrangement 54 via a conduit of the outer balloon 59a or the inner balloon 59b. The cryogen may also be used to pressurize the outer balloon 59a or another fluid may be used, such as saline.

In some embodiments, the outer balloon 59a may have a generally cylindrical outer profile. In other embodiments, the profile of the outer balloon 59a may have a fluted, wave, or other complex shape that is configured to contact a vessel's inner wall at longitudinally and circumferentially spaced-apart locations. Each of these contact locations of the outer balloon 59a preferably incorporates a treatment pattern segment or segments, and the effective coverage area (e.g., area of pattern structure or void) of the treatment pattern segments preferably completes at least one revolution or turn of the outer balloon 59a.

According to other embodiments, as shown in Figure 13B, the outer balloon 59a of the treatment element 50 incorporates a treatment pattern 54 comprising voids or apertures 54a. An inner balloon 59b incorporates a thermally active treatment pattern 59c that is shown to be in alignment with the voids or apertures 54a of the outer balloon 59a. The inner balloon 59b also serves as a biasing balloon that, when inflated, expands and forces at least the treatment pattern 59c of the inner balloon 59a against or in proximity with the inner wall of the target vessel. The inner balloon 59b may be controllably pressurized using saline or the cryogen that is fluidly or thermally coupled to the thermally active treatment pattern 59c. The outer balloon 59a may be controllably pressurized using saline or other passive fluid.

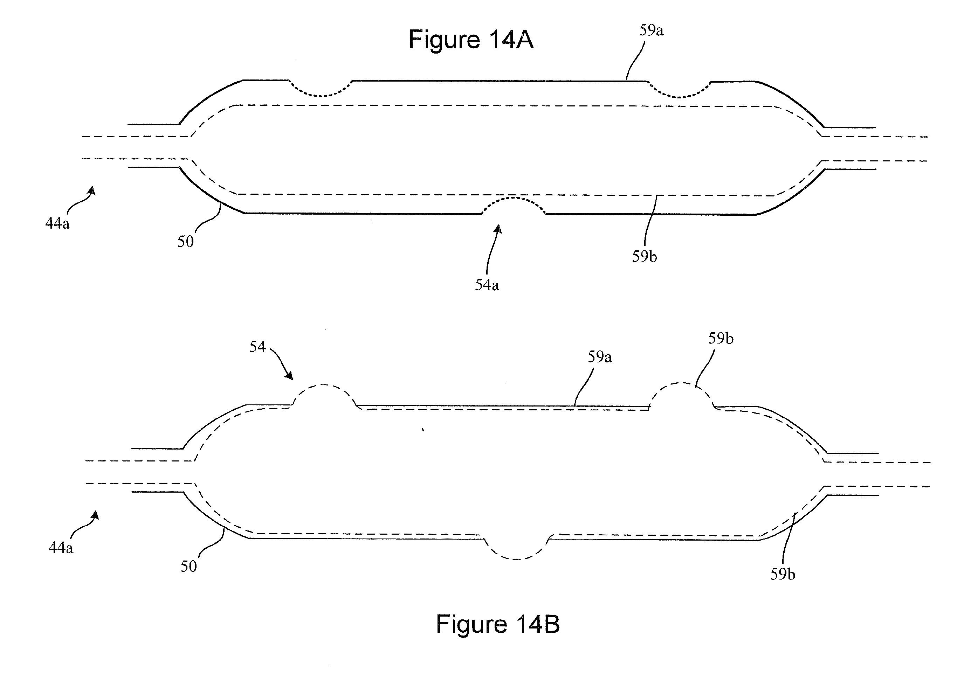

Figures 14A and 14B illustrate a treatment element 50 that includes dual balloon arrangements 59a, 59b. Figure 14A shows the inner balloon 59b in an non-inflated configuration, while Figure 14B shows the inner balloon 59b in an inflated configuration. In this arrangement, the outer balloon 15a has a stiffness greater than that of the inner balloon 59b. The outer balloon 15a, for example, may be a non-compliant balloon and the inner balloon 59b may be a compliant balloon, as are known in the art. The outer balloon 59a includes a treatment pattern 54 comprising a multiplicity of apertures 54a of sufficient size to accommodate a bulged portion of the inner balloon 59b when the inner balloon 59b is pressurized, as is shown in Figure 14B. The effective coverage area of the apertures 54a preferably completes at least one revolution or turn of the outer balloon 59a.

According to one denervation therapy delivery approach, the dual balloon treatment element 50 illustrated in Figures 14A and 14B is advanced in an non-inflated configuration via an intravascular access path to a target vessel, such as the renal artery. After being properly positioned within the renal artery, the outer balloon 59a is inflated such that at least portions of the exterior surface of the outer balloon 59a contact the inner wall of the renal artery. The inner balloon 59b is pressurized so that the exterior wall portions of the inner balloon 59b adjacent the apertures 54a of the outer balloon 59a bulge at least partially into the apertures 54a. In some configurations, it may be desirable that the inner balloon portions adjacent the apertures 54a of the outer balloon 59a expand through the apertures 54a and extend beyond the outer surface of the outer balloon 59a, as is shown in Figure 14B. In other configurations, it may be desirable that the inner balloon portions adjacent the apertures 54a of the outer balloon 59a expand only partially through the apertures 54a, but do not extend beyond the outer surface of the outer balloon 59a.

A cryogen may be used to pressurize the inner balloon 59b which causes thermal cooling/freezing of renal artery tissue in contact with or located proximate to the bulged portions of the inner balloon 59b. The outer balloon structure surrounding the apertures 54a provides a desired degree of thermal insulation so that tissue surrounding the treatment sites experience reduced or negligible injury.

In another configuration, the outer balloon 59a may include a single slot or multiple slots that alone (in the case of a single slot) or collectively (in the case of multiple slots) completes at least one revolution or turn of the outer balloon 59a. When inflated, portions of the inner balloon 59b bulge at least partially into the slot or slots.

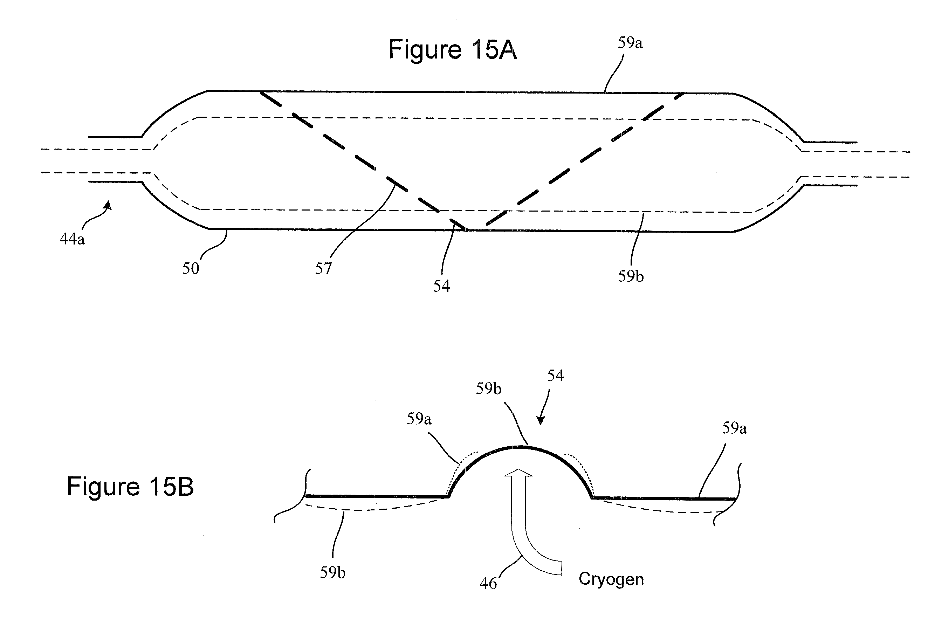

The embodiment illustrated in Figures 15A and 15B has a construction similar to that shown in Figure 13A. As is shown in Figure 15A, the treatment element 50 includes dual balloon arrangements 59a, 59b. In this embodiment, a pre-stressed feature 57 is incorporated into the outer balloon 59a. The pre-stressed feature 57 is configured to separate in response to sufficient tensile force applied to the pre-stressed feature 57. As shown, the pre-stressed 57 feature has a spiral or helical shape. It is understood that other shapes are contemplated, and that the pre-stressed feature 57 need not be a single feature but may comprises multiple pre-stressed features.

As is best seen in Figure 15B, inflation of the inner balloon 59b produces tensile force on the pre-stress feature 57 that causes it to fail at an appropriate level of pressurization. By way of example, the pre-stressed feature 57 splits in response to sufficient tensile force imparted to the outer balloon 59a via pressurization of the inner balloon 59b. Separation of the outer balloon 59a along the pre-stressed feature 57 creates a void into which portions of the inner balloon 59b bulge. The inner balloon 59b is preferably pressurized with a cryogen, which causes thermal cooling/freezing of renal artery tissue in contact with or located proximate to the bulged portions of the inner balloon 59b. The outer balloon structure surrounding the pre-stressed feature 57 provides a desired degree of thermal insulation so that tissue surrounding the treatment sites experience reduced or negligible injury.

It is understood that other types of treatment agents may be delivered using a single or multiple balloon and/or catheter implementation for denervating the renal artery in accordance with embodiments of the invention. For example, a thermal transfer fluid that is heated to a sufficiently high temperature (e.g., greater and 50°C) to disrupt or terminate renal sympathetic nerve activity may be used.

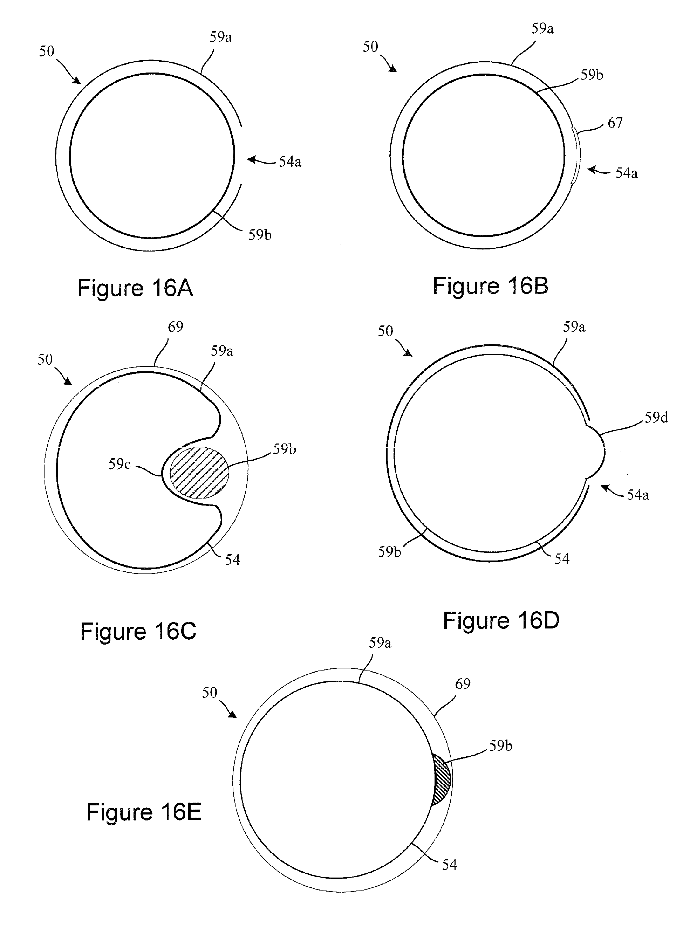

Figures 16A-16E are cross sections of a treatment element 50 in accordance with various embodiments of the present invention. The treatment element embodiment shown in Figure 16A includes an inner balloon 59b partially encompassed by an outer balloon 59a. An aperture 54a is shown provided on the outer balloon 59a. As previously discussed, the inner balloon 59b may be configured as a cryoballoon or a heat therapy delivery balloon, for example. The treatment element 50 shown in Figure 16A may also be representative of a distal end of a treatment catheter, sheath, or sleeve.

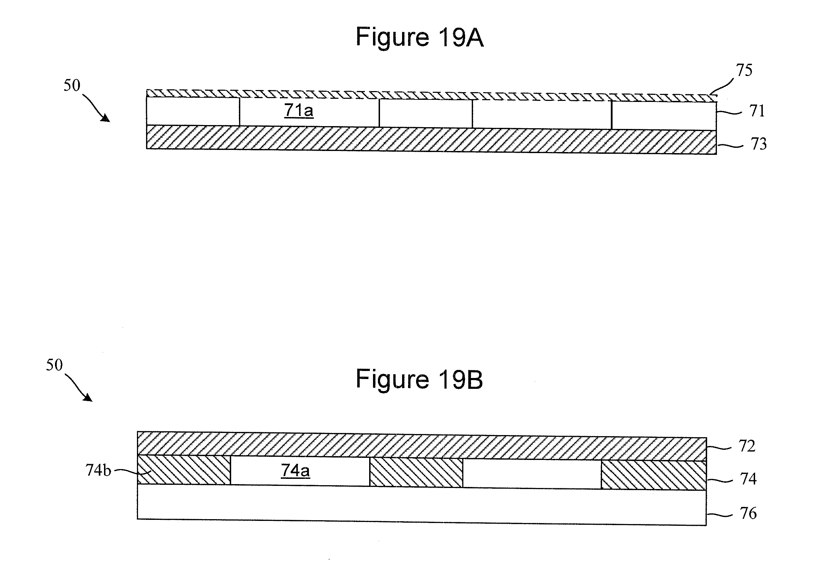

Another view of the treatment element 50 illustrated in Figure 16A is shown in Figure 19A. In the configuration shown in Figure 19A, the treatment element 50 includes an inner layer 73 comprising a material having high thermal conductivity. This inner layer 73 is thermally and/or fluidly coupled to a cryogen source. An outer layer 71 preferably includes a material having low thermal conductivity and serves as thermal insulation. The outer layer 71 is shown to include perforations or apertures 71 a that expose portions of the inner layer 73. The apertures 71a present a low thermal resistance pathway to or through the outer surface of the treatment element 50. In some configurations, the apertures 71a may instead be representative of material with high thermal conductivity, such as a metallic or polymeric composite material. An outer membrane layer 75 may optionally be included and have properties that enhance contact between the treatment element 50 and the inner wall of a target vessel. The outer membrane layer 75 may be fashioned from material having good or neutral thermal conductivity characteristics.

Figure 19B shows another configuration of a treatment element 50 according to embodiments of the invention. The treatment element 50 shown in Figure 19B includes an inner layer 76 comprising a material having low thermal conductivity, such as an insulating material. A middle layer 74 preferably includes a material having high thermal conductivity and is thermally and/or fluidly coupled to a cryogen source. The middle layer 74 may incorporate apertures 74a or material 74a having low thermal conductivity located between portions 74b having high thermal conductivity. An outer layer 72 is preferably formed form a material having high thermal conductivity and contacts the portions 74b of high thermal conductivity of the middle layer 74. An optional outer membrane layer (not shown) may be included and have properties that enhance contact between the treatment element 50 and the inner wall of a target vessel. The outer membrane layer may be fashioned from material having good or neutral thermal conductivity characteristics.

The treatment element configurations shown in Figures 19A and 19B represent some of many possible constructions that provide for effective delivery of thermal denervation therapy to a target vessel. The treatment element configurations shown in Figures 19A and 19B may be incorporated in a variety of catheter, sheath, sleeve, and/or balloon structures. Moreover, the layers of the treatment element configurations shown in Figures 19A and 19B may be distributed on multiple components, such as some layers provided on a balloon and others on a catheter or sleeve configured to mechanically cooperate with the balloon.

The treatment element embodiment shown in Figure 16B is similar to that illustrated in Figure 16A. In Figure 16B, an aperture 54a is shown provided on the outer balloon 59a. A membrane 67 is shown covering the aperture 54a. This membrane 67 is preferably formed from a material having high thermal conductivity or is sufficiently thin to have a negligible affect on the thermal transfer characteristics of the treatment element 50.

In other embodiments, the membrane 67 is formed from a permeable material through which a pharmacological agent or venom may pass. In such embodiments, the inner balloon 59b need not be a cryoballoon, but may instead be configured as a drug eluting balloon.

Figure 16C illustrates an embodiment of a treatment element 50 that includes a biasing balloon 59a and a treatment balloon 59b. The biasing balloon 59a is preferably formed to include a channel or trough 59c having a predefined pattern, such as a spiral, helix or other pattern that preferably completes at least one revolution or turn of the biasing balloon 59a. The biasing balloon 59a may be similar in construction to a dilation balloon. A treatment balloon 59b is sized to be received by the trough 59c of the biasing balloon 59b and extends longitudinally along at least a portion of the trough 59c, preferably along a length sufficient to complete at least one revolution or turn of the biasing balloon 59a. In an other configuration, the treatment balloon 59b represents a lumen that is pressurized by a treatment agent. An outer membrane or sheath 69 preferably encompasses the biasing balloon 59a and the treatment balloon 59b, and is preferably formed of a material with high thermal conductivity, at least for those portions of the outer membrane 69 that are situated adjacent the treatment balloon 59b.

Pressurization of the biasing balloon 59a forces the treatment balloon or lumen 59b to move in a radially outward direction and toward the inner wall of a target vessel. In a configuration that employs a treatment balloon 59b, inflation of the treatment balloon 59b enhances direct or indirect contact between the treatment balloon 59b and the inner wall of the target vessel.

Figure 16D shows a treatment element 50 that includes an outer balloon 59a having a stiffness greater than that of the inner balloon 59b. As was previously discussed, the outer balloon 59a may be a non-compliant balloon and the inner balloon 59b may be a compliant balloon. The outer balloon 59a includes an aperture 54a sized to accommodate a bulged portion 59d of the inner balloon 59b when the inner balloon 59b is pressurized, as is best shown in Figure 14B. The effective coverage area of the pattern of apertures 54a preferably completes at least one revolution or turn of the outer balloon 59a.

Figure 16E illustrates an embodiment of a treatment element 50 that includes a biasing balloon 59a and a treatment balloon 59b formed along an outer wall of the biasing balloon and having a predefined pattern, such as a spiral, helix or other pattern that preferably completes at least one revolution or turn of the biasing balloon 59a. The biasing balloon 59a may be similar in construction to a dilation balloon. In another configuration, the treatment balloon 59b represents a lumen that is pressurized by a treatment agent. An outer membrane or sheath 69 preferably encompasses the biasing balloon 59a and the treatment balloon 59b, and is preferably formed of a material with high thermal conductivity, at least for those portions of the outer membrane 69 that are situated adjacent the treatment balloon 59b. Pressurization of the biasing balloon 59a forces the treatment balloon or lumen 59b to move radially outward toward the inner wall of a target vessel. In a configuration that employs a treatment balloon 59b, inflation of the treatment balloon 59b enhances direct or indirect contact between the treatment balloon 59b and the inner wall of the target vessel.

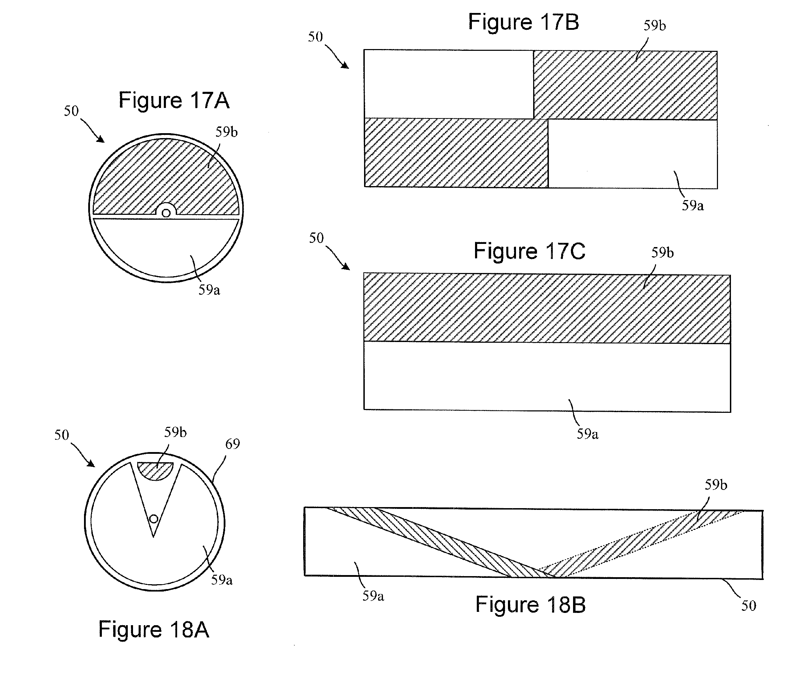

Figures 17A-18B show different embodiments of a multiple lumen catheter configured to deliver denervation therapy to a target vessel in accordance with the present invention. Figure 17A is a cross section of a treatment element 50 provided at the distal end of a multiple lumen catheter. The treatment element 50 of the catheter shown in Figure 17A includes a biasing lumen 59a and a treatment lumen 59b. The treatment lumen 59b has a predefined pattern. When pressurized, the biasing lumen 59a forces the treatment lumen 59b to move radially outward toward the inner wall of a target vessel. An outer sheath 69 at the catheter's distal end preferably encompasses the biasing lumen 59a and the treatment lumen 59b, and is preferably formed of a material with high thermal conductivity, at least for those portions of the outer sheath 69 that are situated adjacent the treatment lumen 59b.

Figures 17B and 17C show different treatment pattern configurations from a surface view of the catheter illustrated in Figure 17A. The surface view of Figure 17A represents the treatment element portion of the catheter's distal end as if it were separated longitudinally and then flattened. In Figure 17B, the treatment pattern includes two rectangular shaped treatment pattern segments 59b which each take on a U- or C-shape when in a cylindrical or tube configuration. The two treatment pattern segments 59b are shown thermally and/or fluidly coupled, but may be separate structures, as in the case of the embodiment shown in Figure 10F. The treatment pattern segments 59b shown in Figure 17B are preferably of sufficient size so that together they complete at least one revolution or turn of the catheter's distal end.

Figure 17C shows a single rectangular treatment pattern 59b which takes on a single U- or C-shape when in a cylindrical or tube configuration. Unlike the embodiment shown in Figure 17B, the treatment pattern 59b of Figure 17C is not of sufficient size to complete at least one revolution or turn of the catheter's distal end. Rather, the treatment pattern 59b of Figure 17C is configured to treat less than the full circumference of the inner wall of a target vessel. Denervation therapy delivery of a target vessel using a treatment pattern 59b of the type depicted in Figure 17C finds particular usefulness when partial circumferential denervation of a target vessel is desired with the treatment element 50 positioned at a particular vessel location. Should complete circumferential denervation of the target vessel be desired, the catheter's distal end may be rotated and preferably longitudinally displaced relative to the prior treatment element location within the vessel to complete the denervation therapy. It is understood that a treatment pattern have a shape or size insufficient to complete a full revolution or turn of the treatment element may be incorporated in or on a balloon arrangement in accordance with embodiments of the invention.

The treatment element 50 of the catheter shown in Figure 18A includes a biasing lumen 59a and a treatment lumen 59b. The treatment lumen 59b has a predefined pattern, such as a spiral, helix or other pattern, that preferably completes at least one revolution or turn of the catheter's distal end, as is best see in Figure 18B. When pressurized, the biasing lumen 59a forces the treatment lumen 59b to move radially outward toward the inner wall of a target vessel. An outer sheath 69 at the catheter's distal end preferably encompasses the biasing lumen 59a and the treatment lumen 59b, and is preferably formed of a material with high thermal conductivity, at least for those portions of the outer sheath 69 that are situated adjacent the treatment lumen 59b.

Embodiments of the present invention may be implemented to provide varying degrees of denervation therapy to innervated renal vasculature. For example, embodiments of the present invention may provide for control of the extent and relative permanency of renal nerve impulse transmission interruption achieved by denervation therapy delivered using a treatment apparatus of the present invention. The extent and relative permanency of renal nerve injury may be tailored to achieve a desired reduction in sympathetic nerve activity (including a partial or complete block) and to achieve a desired degree of permanency (including temporary or irreversible injury).

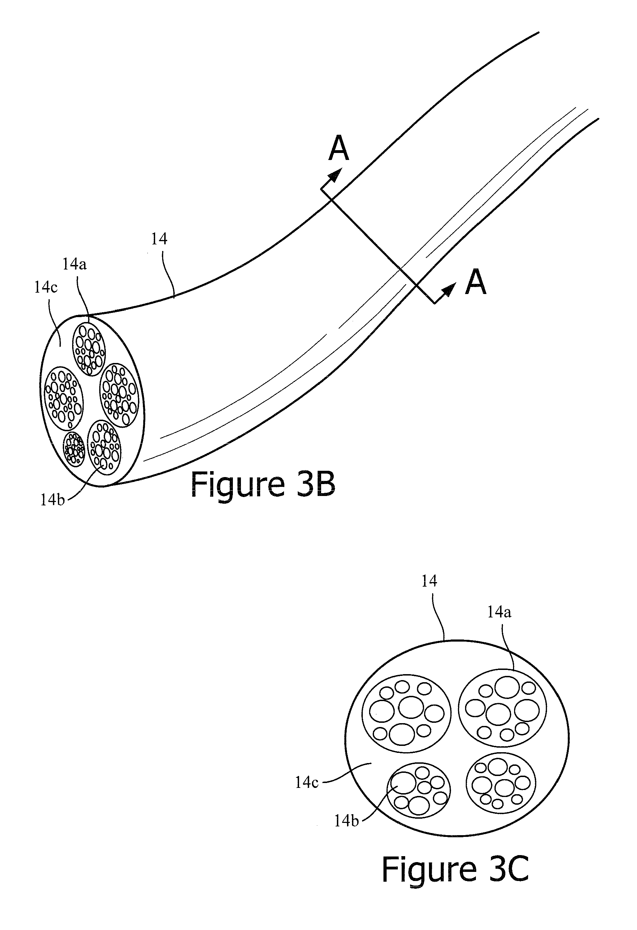

Returning to Figures 3B and 3C, the portion of the renal nerve 14 shown in Figures 3B and 3C includes bundles 14a of nerve fibers 14b each comprising axons or dendrites that originate or terminate on cell bodies or neurons located in ganglia or on the spinal cord, or in the brain. Supporting tissue structures 14c of the nerve 14 include the endoneurium (surrounding nerve axon fibers), perineurium (surrounds fiber groups to form a fascicle), and epineurium (binds fascicles into nerves), which serve to separate and support nerve fibers 14b and bundles 14a. In particular, the endoneurium, also referred to as the endoneurium tube or tubule, is a layer of delicate connective tissue that encloses the myelin sheath of a nerve fiber 14b within a fasciculus.

Major components of a neuron include the soma, which is the central part of the neuron that includes the nucleus, cellular extensions called dendrites, and axons, which are cable-like projections that carry nerve signals. The axon terminal contains synapses, which are specialized structures where neurotransmitter chemicals are released in order to communicate with target tissues. The axons of many neurons of the peripheral nervous system are sheathed in myelin, which is formed by a type of glial cell known as Schwann cells. The myelinating Schwann cells are wrapped around the axon, leaving the axolemma relatively uncovered at regularly spaced nodes, called nodes of Ranvier. Myelination of axons enables an especially rapid mode of electrical impulse propagation called saltation.

In some embodiments, a treatment apparatus of the present invention may be implemented to deliver denervation therapy that causes transient and reversible injury to renal nerve fibers 14b. In other embodiments, a treatment apparatus of the present invention may be implemented to deliver denervation therapy that causes more severe injury to renal nerve fibers 14b, which may be reversible if the therapy is terminated in a timely manner. In preferred embodiments, a treatment apparatus of the present invention may be implemented to deliver denervation therapy that causes severe and irreversible injury to renal nerve fibers 14b, resulting in permanent cessation of renal sympathetic nerve activity. For example, a treatment apparatus may be implemented to deliver a denervation therapy that disrupts nerve fiber morphology to a degree sufficient to physically separate the endoneurium tube of the nerve fiber 14b, which can prevent regeneration and re-innervation processes.

By way of example, and in accordance with Seddon's classification as is known in the art, a treatment apparatus of the present invention may be implemented to deliver a denervation therapy that interrupts conduction of nerve impulses along the renal nerve fibers 14b by imparting damage to the renal nerve fibers 14b consistent with neruapraxia. Neurapraxia describes nerve damage in which there is no disruption of the nerve fiber 14b or its sheath. In this case, there is an interruption in conduction of the nerve impulse down the nerve fiber, with recovery taking place within hours to months without true regeneration, as Wallerian degeneration does not occur. Wallerian degeneration refers to a process in which the part of the axon separated from the neuron's cell nucleus degenerates. This process is also known as anterograde degeneration. Neurapraxia is the mildest form of nerve injury that may be imparted to renal nerve fibers 14b by use of a treatment apparatus according to embodiments of the present invention.

A treatment apparatus may be implemented to interrupt conduction of nerve impulses along the renal nerve fibers 14b by imparting damage to the renal nerve fibers consistent with axonotmesis. Axonotmesis involves loss of the relative continuity of the axon of a nerve fiber and its covering of myelin, but preservation of the connective tissue framework of the nerve fiber. In this case, the encapsulating support tissue 14c of the nerve fiber 14b are preserved. Because axonal continuity is lost, Wallerian degeneration occurs. Recovery from axonotmesis occurs only through regeneration of the axons, a process requiring time on the order of several weeks or months. Electrically, the nerve fiber 14b shows rapid and complete degeneration. Regeneration and re-innervation may occur as long as the endoneural tubes are intact.

A treatment apparatus may be implemented to interrupt conduction of nerve impulses along the renal nerve fibers 14b by imparting damage to the renal nerve fibers 14b consistent with neurotmesis. Neurotmesis, according to Seddon's classification, is the most serious nerve injury in the scheme. In this type of injury, both the nerve fiber 14b and the nerve sheath are disrupted. While partial recovery may occur, complete recovery is not possible. Neurotmesis involves loss of continuity of the axon and the encapsulating connective tissue 14c, resulting in a complete loss of autonomic function, in the case of renal nerve fibers 14b. If the nerve fiber 14b has been completely divided, axonal regeneration causes a neuroma to form in the proximal stump.

A more stratified classification of neurotmesis nerve damage may be found by reference to the Sunderland System as is known in the art. The Sunderland System defines five degrees of nerve damage, the first two of which correspond closely with neurapraxia and axonotmesis of Seddon's classification. The latter three Sunderland System classifications describe different levels of neurotmesis nerve damage.

The first and second degrees of nerve injury in the Sunderland system are analogous to Seddon's neurapraxia and axonotmesis, respectively. Third degree nerve injury, according to the Sunderland System, involves disruption of the endoneurium, with the epineurium and perineurium remaining intact. Recovery may range from poor to complete depending on the degree of intrafascicular fibrosis. A fourth degree nerve injury involves interruption of all neural and supporting elements, with the epineurium remaining intact. The nerve is usually enlarged. Fifth degree nerve injury involves complete transection of the nerve fiber 14b with loss of continuity.

As discussed above in accordance with various embodiments, denervation therapy may be delivered to innervated renal vasculature using a treatment arrangement that incorporates a cryoballoon 50. Renal denervation therapy may be controlled to achieve a desired degree of attenuation in renal nerve activity in accordance with embodiments of the present invention. Selecting or controlling cryotherapy delivered by a cryoballoon catheter of the present invention advantageously facilitates experimentation and titration of a desired degree and permanency of renal sympathetic nerve activity cessation.

For example, renal nerve fiber regeneration and re-innervation may be permanently compromised by applying cryogenic therapy to innervated renal vasculature, including the ostium of the renal artery and renal ganglia, at a sufficiently low temperature to allow ice crystals to form inside nerve fibers 14b. Formation of ice crystals inside nerve fibers 14b of innervated renal arterial tissue and renal ganglia tears the nerve cells apart, and physically disrupts or separates the endoneurium tube, which can prevent regeneration and re-innervation processes. Delivery of cryogenic therapy to renal nerves 14 at a sufficiently low temperature in accordance with embodiments of the present invention can cause necrosis of renal nerve fibers 14b, resulting in a permanent and irreversible loss of the conductive function of renal nerve fibers 14b.

In general, embodiments of a cryoballoon catheter of the present invention may be implemented to deliver cryogenic therapy to cause renal denervation at therapeutic temperatures ranging between approximately 0°C and approximately -180°C. For example, embodiments of a cryoballoon catheter may be implemented to deliver cryogenic therapy to cause renal denervation with temperatures at the renal nerves ranging from approximately 0°C to approximately -30°C at the higher end, and to about -140°C to - 180°C at the lower end. Less robust renal nerve damage is likely for temperatures approaching and greater than 0°C, and more robust acute renal denervation is likely for temperatures approaching and less than -30°C, for example, down to -120C to -180C. These therapeutic temperature ranges may be determined empirically for a patient, a patient population, or by use of human or other mammalian studies.

It has been found that delivering cryotherapy to the renal artery and the renal ganglia at a sufficiently low temperature with freeze/thaw cycling allows ice crystals to form inside nerve fibers 14b and disrupt renal nerve function and morphology. For example, achieving therapeutic temperatures that range from -30°C to +10°C at a renal nerve for treatment times of 30 seconds to 4 minutes and thaw times of about 1 to 2 minutes has been found to cause acute renal denervation in at least some of the renal nerves in a porcine model.

The representative embodiments described below are directed to cryocatheter apparatuses that can be configured for delivering cryogenic therapy to renal vasculature at specified therapeutic temperatures or temperature ranges, causing varying degrees of nerve fiber degradation. As was discussed above, therapeutic temperature ranges achieved by cryocatheters (e.g., cryoballoon catheters) of the present invention may be determined using non-human mammalian studies. The therapeutic temperatures and degrees of induced renal nerve damage described in the context of the following embodiments are based largely on cryoanalgesia studies performed on rabbits (*see, e.g.,* L. Zhou et al., Mechanism Research of Cryoanalgesia, Neurological Research, Vol. 17, pp. 307-311 (1995)), but may generally be applicable for human renal vasculature. As is discussed below, the therapeutic temperatures and degrees of induced renal nerve damage may vary somewhat or significantly from those described in the context of the following embodiments based on a number of factors, including the design of the cryotherapy apparatus, duration of cryotherapy, and the magnitude of mechanical disruption of nerve fiber structure that can be achieved by subjecting renal nerves to freeze/thaw cycling, among others.

In accordance with various embodiments, a cryoballoon catheter of the present invention may be implemented to deliver cryogenic therapy to cause a minimum level of renal nerve damage. Cooling renal nerve fibers to a therapeutic temperature ranging between about 0°C and about -20°C is believed sufficient to temporarily block some or all renal sympathetic nerve activity and cause a minimum degree of renal nerve damage, consistent with neurapraxia for example. Freezing renal nerves to a therapeutic temperature of -20°C or higher may not cause a permanent change in renal nerve function or morphology. At therapeutic temperatures of -20°C or higher, slight edema and myelin swelling may occur in some of the renal nerve fibers, but these conditions may be resolved after thawing.

In other embodiments, cooling renal nerve fibers to a therapeutic temperature ranging between about -20°C and about -60°C is believed sufficient to block all renal sympathetic nerve activity and cause an intermediate degree of renal nerve damage, consistent with axonotmesis (and possibly some degree of neurotmesis for lower temperatures of the -20°C and -60°C range), for example. Cooling renal nerves to a therapeutic temperature of -60°C may cause freezing degeneration and loss of renal nerve conductive function, but may not result in a permanent change in renal nerve function or morphology. However, renal nerve regeneration is substantially slowed (e.g., on the order of 90 days). At a therapeutic temperature of -60°C, the frozen renal nerve is likely to demonstrate edema with thickening and loosening of the myelin sheaths and irregular swelling of axons, with Schwann cells likely remaining intact.

In further embodiments, cooling renal nerve fibers to a therapeutic temperature ranging between about -60°C and about -100°C is believed sufficient to block all renal sympathetic nerve activity and cause an intermediate to a high degree of renal nerve damage, consistent with neurotmesis, for example. Cooling renal nerves to a therapeutic temperature of -100°C, for example, causes swelling, thickening, and distortion in a large percentage of axons. Exposing renal nerves to a therapeutic temperature of -100°C likely causes splitting or focal necrosis of myelin sheaths, and microfilament, microtubular, and mitochondrial edema. However, at a therapeutic temperature of -100°C, degenerated renal nerves may retain their basal membranes, allowing for complete recovery over time. Although substantially slowed (e.g., on the order of 180 days), renal nerve regeneration may occur and be complete.

In accordance with other embodiments, cooling renal nerve fibers to a therapeutic temperature of between about -140°C and about -180°C is believed sufficient to block all renal sympathetic nerve activity and cause a high degree of renal nerve damage, consistent with neurotmesis for example. Application of therapeutic temperatures ranging between about -140°C and about -180°C to renal nerve fibers causes immediate necrosis, with destruction of basal membranes (resulting in loss of basal laminea scaffolding needed for complete regeneration). At these low temperatures, axoplasmic splitting, axoplasmic necrosis, and myelin sheath disruption and distortion is likely to occur in most renal nerve fibers. Proliferation of collagen fibers is also likely to occur, which restricts renal nerve regeneration.

It is believed that exposing renal nerves to a therapeutic temperature of about -140°C or lower causes permanent, irreversible damage to the renal nerve fibers, thereby causing permanent and irreversible termination of renal sympathetic nerve activity. For some patients, exposing renal nerves to a therapeutic temperature ranging between about - 120°C and about -140°C may be sufficient to provide similar permanent and irreversible damage to the renal nerve fibers, thereby causing permanent and irreversible cessation of renal sympathetic nerve activity. In other patients, it may be sufficient to expose renal nerves to a therapeutic temperature of at least -30°C in order to provide a desired degree of renal sympathetic nerve activity cessation.

In preferred embodiments, it is desirable that the cryogen used to deliver cryotherapy to renal vasculature be capable of freezing target tissue so that nerve fibers innervating the renal artery 12 are irreversibly injured, such that nerve conduction along the treated renal nerve fibers is permanently terminated. Suitable cryogens include those capable of cooling renal nerve fibers and renal ganglia to temperatures of at least about - 120°C or lower, preferably to temperatures of at least about -130°C or lower, and more preferably to temperatures of at least about -140°C or lower. It is understood that use of cryogens that provide for cooling of renal nerve fibers and renal ganglia to temperatures of at least about -30°C may effect termination of renal sympathetic nerve activity with varying degrees of permanency.

The temperature ranges and associated degrees of induced renal nerve damage described herein are provided for non-limiting illustrative purposes. Actual therapeutic temperatures and magnitudes of resulting nerve injury may vary somewhat or significantly from those described herein, and be impacted by a number of factors, including patient-specific factors (e.g., the patient's unique renal vasculature and sympathetic nervous system characteristics), therapy duration, frequency and duration of freeze/thaw cycling, structural characteristics of the cryotherapy catheter and/or balloon arrangement, type of cryogen used, and method of delivering cryotherapy, among others.

It is believed that higher degrees of renal nerve injury may be achieved by subjecting renal nerves to both cryotherapy and freeze/thaw cycling when compared to delivering cryotherapy without employing freeze/thaw cycling. Implementing freeze/thaw cycling as part of cryotherapy delivery to renal nerves may result in achieving a desired degree of renal sympathetic nerve activity attenuation (e.g., termination) and permanency (e.g., irreversible) at therapeutic temperatures higher than those discussed above. Various thermal cycling parameters may be selected for, or modified during, renal denervation cryotherapy to achieve a desired level of renal nerve damage, such parameters including the number of freeze/thaw cycles, high and low temperature limits for a given freeze/thaw cycle, the rate of temperature change for a given freeze/thaw cycle, and the duration of a given freeze/thaw cycle, for example. As was previously discussed, these therapeutic temperature ranges and associated degrees of induced renal nerve damage may be determined empirically for a particular patient or population of patients, or by use of human or other mammalian studies.

The foregoing description of the various embodiments of the invention has been presented for the purposes of illustration and description. It is not intended to be exhaustive or to limit the invention to the precise form disclosed. Many modifications and variations are possible in light of the above teaching. It is intended that the scope of the invention be limited not by this detailed description, but rather by the claims appended hereto.

## Claims

1. A catheter system, comprising:
a catheter comprising a flexible shaft having a proximal end, a distal end, a length, and a lumen arrangement extending between the proximal and distal ends, the length of the shaft sufficient to access a patient's renal artery relative to a percutaneous access location of the patient;
a thermal transfer fluid source; and
a treatment element provided at the distal end of the shaft and fluidly coupled to the lumen arrangement, the treatment element dimensioned for deployment on or within the renal artery and comprising a predefined patterned treatment surface including one or more treatment pattern portions and one or more thermal insulation portions, the one or more treatment pattern portions arranged to complete at least one revolution of the treatment element, the treatment element configured to receive a thermal transfer fluid from the thermal transfer fluid source through the lumen arrangement and deliver thermal denervation therapy from the thermal transfer fluid via the one or more treatment pattern portions to one or more regions of the renal artery adjacent the one or more treatment pattern portions such that at least one complete revolution of the renal artery is subjected to the thermal denervation therapy.

2. The system according to claim 1, wherein the catheter comprises a hinge mechanism provided on the shaft proximal of the treatment element, the hinge mechanism configured to facilitate preferential bending at the distal end of the shaft to aid in directing the treatment element into the renal artery from the abdominal aorta, wherein the hinge mechanism comprises a slotted tube arrangement.

3. The apparatus according to claim 1 or 2, comprising a biasing mechanism configured to generate a biasing force sufficient to effect engagement between the one or more treatment pattern portions of the treatment element and the inner wall of the renal artery.

4. The apparatus according to claim 1 through claim 3, wherein the one or more treatment pattern portions are formed as an integral part of the distal end of the shaft.

5. The apparatus according to claim 1 through claim 3, wherein the one or more treatment pattern portions are formed as a sleeve, and the sleeve is mounted at the distal end of the shaft.

6. The apparatus according to either of claim 4 and claim 5, comprising a cryoballoon fluidly coupled to the lumen arrangement of the shaft and the lumen arrangement is configured to receive the cryoballoon, wherein pressurizing the cryoballoon expands the one or more treatment pattern portions and causes the one or more treatment pattern portions to contact or come into close proximity of an inner wall of the renal artery.

7. The apparatus according to claim 6, wherein the distal end of the shaft is formed from a material having a stiffness greater than a stiffness of the cryoballoon, the relative stiffness of the shaft's distal end and the cryoballoon material allowing those portions of the cryoballoon's exterior surface exposed by apertures of the one or more treatment pattern portions to expand into the apertures and contact the inner wall of the renal artery.

8. The apparatus according to any of claim 1 through claim 5, wherein the treatment element comprises a cryoballoon fluidly coupled to the lumen arrangement of the shaft, and the one or more treatment pattern portions are formed as an integral part of the cryoballoon.

9. The apparatus according to any of claim 1 through claim 5, wherein the treatment element comprises a cryoballoon fluidly coupled to the lumen arrangement of the shaft, and the one or more treatment pattern portions are formed as a separate sleeve affixed to an outer surface of the cryoballoon.

10. The apparatus according to any of claim 1 through claim 9, wherein the one or more treatment pattern portions comprise a predetermined pattern of apertures.

11. The apparatus according to any of claim 1 through claim 9, wherein the one or more treatment pattern portions comprise a predetermined pattern of thermally conductive elements or regions.

12. The apparatus according to any of claim 1 through claim 11, wherein the one or more treatment pattern portions comprise a generally spiral or helical pattern.

13. The apparatus according to any of claim 1 through claim 12, wherein the one or more treatment pattern portions comprise a generally longitudinal and contiguous pattern, or wherein the one or more treatment pattern portions comprise a generally longitudinal and non-contiguous pattern.

14. The apparatus according to any of claim 1 through claim 13, wherein the thermal transfer fluid comprises a cryogen having a boiling temperature at ambient pressure of about 0°C or lower.

## Patentansprüche

1. Kathetersystem, das aufweist:
einen Katheter, der einen flexiblen Schaft mit einem proximalen Ende, einem distalen Ende, einer Länge und einer Lumenanordnung aufweist, die sich zwischen dem proximalen und distalen Ende erstreckt, wobei die Länge des Schafts ausreichend ist, um relativ zu einer perkutanen Zugangsstelle Zugang zur Nierenarterie eines Patienten zu erlangen;
eine Wärmeübertragungsfluidquelle; und
ein Behandlungselement, das am distalen Ende des Schafts vorgesehen ist und fluidisch mit der Lumenanordnung gekoppelt ist, wobei das Behandlungselement zum Einsatz an oder innerhalb der Nierenarterie bemessen ist und eine vordefinierte gemusterte Behandlungsfläche aufweist, die einen oder mehrere Behandlungsmusterabschnitte und einen oder mehrere Wärmeisolationsabschnitte aufweist, der eine oder die mehreren Behandlungsmusterabschnitte eingerichtet sind, mindestens eine Umdrehung des Behandlungselements zu vollenden, das Behandlungselement konfiguriert ist, ein Wärmeübertragungsfluid von der Wärmeübertragungsfluidquelle durch die Lumenanordnung aufzunehmen und eine thermische Denervierungstherapie vom Wärmeübertragungsfluid über den einen oder die mehreren Behandlungsmusterabschnitte einem oder mehreren Bereichen der Nierenarterie zu verabreichen, die dem einen oder den mehreren Behandlungsmusterabschnitten benachbart sind, so dass mindestens eine vollständige Umdrehung der Nierenarterie der thermischen Denervierungstherapie unterzogen wird.

2. System nach Anspruch 1, wobei der Katheter einen Gelenkmechanismus aufweist, der am Schaft proximal vom Behandlungselement vorgesehen ist, wobei der Gelenkmechanismus konfiguriert ist, eine bevorzugte Biegung am distalen Ende des Schafts zu erleichtern, um die Führung des Behandlungselements von der Bauchaorta in die Nierenarterie zu unterstützen, wobei der Gelenkmechanismus eine Schlitzröhrenanordnung aufweist.

3. Vorrichtung nach Anspruch 1 oder 2, die einen Vorspannmechanismus aufweist, der konfiguriert ist, eine Vorspannkraft zu erzeugen, die ausreicht, einen Eingriff zwischen dem einen oder den mehreren Behandlungsmusterabschnitten des Behandlungselements und der Innenwand der Nierenarterie zu bewirken.

4. Vorrichtung nach Anspruch 1 bis 3, wobei der eine oder die mehreren Behandlungsmusterabschnitte als ein integraler Teil des distalen Endes des Schafts ausgebildet sind.

5. Vorrichtung nach Anspruch 1 bis 3, wobei der eine oder die mehreren Behandlungsmusterabschnitte als eine Hülse ausgebildet sind und die Hülse am distalen Ende des Schafts angebracht ist.

6. Vorrichtung nach einem der Ansprüche 4 und 5, die einen Kryoballon aufweist, der fluidisch mit der Lumenanordnung des Schafts gekoppelt ist, und die Lumenanordnung konfiguriert ist, den Kryoballon aufzunehmen, wobei eine Druckbeaufschlagung des Kryoballons den einen oder die mehreren Behandlungsmusterabschnitte ausdehnt und bewirkt, dass der eine oder die mehreren Behandlungsmusterabschnitte eine Innenwand der Nierenarterie berühren oder in deren unmittelbare Nähe gelangen.

7. Vorrichtung nach Anspruch 6, wobei das distale Ende des Schafts aus einem Material ausgebildet ist, das eine Steifigkeit aufweist, die größer als eine Steifigkeit des Kryoballons ist, wobei die relative Steifigkeit des distalen Endes des Schafts und des Kryoballonmaterials es ermöglicht, dass jene Abschnitte der Außenfläche des Kryoballons, die durch Öffnungen des einen oder der mehreren Behandlungsmusterabschnitte freiliegen, sich in die Öffnungen ausdehnen und die Innenwand der Nierenarterie berühren.

8. Vorrichtung nach einem der Ansprüche 1 bis 5, wobei das Behandlungselement einen Kryoballon aufweist, der fluidisch mit der Lumenanordnung des Schafts gekoppelt ist, und der eine oder die mehreren Behandlungsmusterabschnitte als ein integraler Teil des Kryoballons ausgebildet sind.

9. Vorrichtung nach einem der Ansprüche 1 bis 5, wobei das Behandlungselement einen Kryoballon aufweist, der fluidisch mit der Lumenanordnung des Schafts gekoppelt ist, und der eine oder die mehreren Behandlungsmusterabschnitte als eine getrennte Hülse ausgebildet sind, die an einer Außenfläche des Kryoballons befestigt ist.

10. Vorrichtung nach einem der Ansprüche 1 bis 9, wobei der eine oder die mehreren Behandlungsmusterabschnitte ein vorgegebenes Muster von Öffnungen aufweisen.

11. Vorrichtung nach einem der Ansprüche 1 bis 9, wobei der eine oder die mehreren Behandlungsmusterabschnitte ein vorgegebenes Muster von wärmeleitfähigen Elementen oder Bereichen aufweisen.

12. Vorrichtung nach einem der Ansprüche 1 bis 11, wobei der eine oder die mehreren Behandlungsmusterabschnitte ein im Allgemeinen spiralförmiges oder schraubenförmiges Muster aufweisen.

13. Vorrichtung nach einem der Ansprüche 1 bis 12, wobei der eine oder die mehreren Behandlungsmusterabschnitte ein im Allgemeinen longitudinales und zusammenhängendes Muster aufweisen, oder wobei der eine oder die mehreren Behandlungsmusterabschnitte ein im Allgemeinen longitudinales und nicht zusammenhängendes Muster aufweisen.

14. Vorrichtung nach einem der Ansprüche 1 bis 13, wobei das Wärmeübertragungsfluid ein Kältemittel aufweist, das eine Siedetemperatur bei Umgebungsdruck von etwa 0°C oder niedriger aufweist.

## Revendications

1. Système de cathéter, comprenant :
un cathéter comportant une tige flexible avec une extrémité proximale, une extrémité distale, une longueur, et une unité de lumière s'étendant entre l'extrémité proximale et l'extrémité distale, la longueur de la tige étant suffisante pour accéder à une artère rénale d'un patient par rapport à un site d'accès percutané du patient ;
une source de fluide de transfert thermique ; et
un élément de traitement prévu à l'extrémité distale de la tige et en liaison fluidique avec l'unité de lumière, l'élément de traitement étant dimensionné pour être déployé sur ou à l'intérieur de l'artère rénale et présentant une surface de traitement à configuration prédéfinie comportant une ou plusieurs sections de configuration de traitement et une ou plusieurs sections d'isolation thermique, la ou les sections de configuration de traitement étant disposées de manière à effectuer au moins une révolution de l'élément de traitement, l'élément de traitement étant prévu pour recevoir un fluide de transfert thermique de la source de fluide de transfert thermique par l'unité de lumière et administrer par l'intermédiaire de la ou des sections de configuration de traitement un traitement thermique de dénervation par le fluide de transfert thermique à une ou plusieurs régions de l'artère rénale adjacentes à la ou aux sections de configuration de traitement, de manière à soumettre au traitement thermique de dénervation au moins une révolution complète de l'artère rénale.

2. Système selon la revendication 1, où le cathéter comprend un mécanisme à charnière prévu sur la tige proximalement à l'élément de traitement, ledit mécanisme à charnière étant prévu pour faciliter une flexion préférentielle à l'extrémité distale de la tige pour assister le guidage de l'élément de traitement dans l'artère rénale depuis l'aorte abdominale, le mécanisme à charnière comportant une unité de tube fendu.

3. Système selon la revendication 1 ou la revendication 2, comprenant un mécanisme de contrainte prévu pour générer une force de contrainte suffisante pour réaliser un contact entre la ou les sections de configuration de traitement de l'élément de traitement et la paroi intérieure de l'artère rénale.

4. Système selon les revendications 1 à 3, où la ou les sections de configuration de traitement sont formées comme partie intégrante de l'extrémité distale de la tige.

5. Système selon les revendications 1 à 3, où la ou les sections de configuration de traitement sont formées comme un manchon, et où ledit manchon est monté à l'extrémité distale de la tige.

6. Système selon la revendication 4 ou la revendication 5, comprenant un cryoballon en liaison fluidique avec l'unité de lumière de la tige et où l'unité de lumière est prévue pour recevoir le cryoballon, la pressurisation du cryoballon expansant la ou les sections de configuration de traitement et entraînant la mise en contact de la ou des sections de configuration de traitement avec une paroi intérieure de l'artère rénale, ou leur positionnement à proximité immédiate de celle-ci.

7. Système selon la revendication 6, où l'extrémité distale de la tige est constituée d'un matériau présentant une rigidité supérieure à la rigidité du cryoballon, la rigidité relative de l'extrémité distale de la tige et du matériau du cryoballon permettant aux parties de la surface extérieure du cryoballon exposées à des ouvertures de la ou des sections de configuration de traitement de s'expanser dans les ouvertures et de contacter la paroi intérieure de l'artère rénale.

8. Système selon l'une des revendications 1 à 5, où l'élément de traitement comprend un cryoballon en liaison fluidique avec l'unité de lumière de la tige, et où la ou les sections de configuration de traitement sont formées comme partie intégrante du cryoballon.

9. Système selon l'une des revendications 1 à 5, où l'élément de traitement comprend un cryoballon en liaison fluidique avec l'unité de lumière de la tige, et où la ou les sections de configuration de traitement sont formées comme un manchon séparé fixé sur une surface extérieure du cryoballon.

10. Système selon l'une des revendications 1 à 9, où la ou les sections de configuration de traitement présentent une configuration d'ouvertures prédéfinie.

11. Système selon l'une des revendications 1 à 9, où la ou les sections de configuration de traitement présentent une configuration prédéfinie d'éléments thermoconducteurs ou de zones thermoconductrices.

12. Système selon l'une des revendications 1 à 11, où la ou les sections de configuration de traitement présentent une configuration sensiblement en spirale ou en hélice.

13. Système selon l'une des revendications 1 à 12, où la ou les sections de configuration de traitement présentent une configuration sensiblement longitudinale et contiguë, ou bien où la ou les sections de configuration de traitement présentent une configuration sensiblement longitudinale et non contiguë.

14. Système selon l'une des revendications 1 à 13, où le fluide de transfert thermique contient un cryogène ayant une température d'ébullition égale ou inférieure à près de 0° C à pression ambiante.
